(19)

Europäisches
Patentamt
European
Patent Office
Office européen
des brevets

(11) **EP 3 696 822 A1**

(12) **EUROPEAN PATENT APPLICATION**

(43) Date of publication:
**19.08.2020 Bulletin 2020/34**

(21) Application number: **19157826.9**

(22) Date of filing: **18.02.2019**

(51) Int Cl.:
**G16H 50/20** (2018.01) **G16B 40/00** (2019.01)
**G16B 20/20** (2019.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **metanomics Health GmbH
10589 Berlin (DE)**

(72) Inventor: **The designation of the inventor has not
yet been filed**

(74) Representative: **Altmann Stößel Dick
Patentanwälte PartG mbB
Dudenstrasse 46
68167 Mannheim (DE)**

(54) **MEANS AND METHODS FOR DETERMINING A PERSONALIZED CUTOFF VALUE FOR A
BIOMARKER**

(57)     The present invention relates to the field of biomarkers and diagnostic methods. Specifically,  the
present invention contemplates a method for determining a personalized cutoff value for a biomarker and a
method for diagnosing a disease which is indicated by a
biomarker. The invention also relates to the use of at
least two metabolic analytes for determining a personalized cutoff value for a biomarker and tools for carrying
out the aforementioned methods, such as analytic and
diagnostic devices.

EP 3 696 822 A1

**Description**

The present invention relates to the field of biomarkers and diagnostic methods. Specifically, the present invention contemplates a method for determining a personalized cutoff value for a biomarker and a method for diagnosing a disease which is indicated by a biomarker. The invention also relates to the use of at least two metabolic analytes for determining a personalized cutoff value for a biomarker and tools for carrying out the aforementioned methods, such as analytic and diagnostic devices.

The early and reliable identification of a disease and the subsequent treatment of patients at an early stage of their disease are of high importance in modern medicine. To this end, diagnostic biomarker assays have been developed that can predict whether a patient is suffering from a disease or progression of a disease or whether a subject has an increased risk to develop a certain disease. However, biomarker assays currently available at the market are often associated with high false positive or false negative rates, hampering their use in clinical diagnostics and affecting the correct treatment of the individual patient.

In general, quantitative diagnostic biomarker tests are used, where a certain cutoff value is given that distinguishes between two test outcomes: positive or negative. However, there is always a certain area in which, depending on the location or definition of the cutoff point, a subset of patients is wrongly classified as test-positive or test-negative. Moreover, the molecular and physiological set-up of an individual patient is typically not considered when performing such an assay with a defined cutoff point.

Since heart disease and cancer are the first and second leading causes of death in the modern world, the development of novel biomarker assays to detect the diseases at an early stage has been a focus of research. Biomarker assays currently available at the market include, for example, NT-proBNP (N-terminal pro-Brain Natriuretic Peptide) biomarker assays with a typical cut-off of 125 pg/ml for the exclusion of heart failure and CA 19-9 (cancer antigen 19-9) biomarker assays with a typical cutoff of 37 U/ml for the diagnosis of pancreatic cancer.

Pancreatic cancer, in particular Pancreatic Ductal Adenocarcinoma (PDAC), is typically one of the cancers with a bad prognosis and a 5-year survival rate of only 0.5-5%. The cancer antigen 19-9 (CA 19-9) was shown to be increased in pancreatic cancer patients and is currently used as biomarker in the diagnosis of PDAC (Fry et al., 2008. Molecular markers of pancreatic cancer: development and clinical relevance. Langenbeck's archives of surgery, 393(6), 883-890.).

CA 19-9 blood levels are elevated in many patients with pancreatic cancer. The CA19-9 level is of limited value for pancreatic cancer diagnostics in terms of both sensitivity and specificity. CA19-9 specificity for pancreatic cancer diagnostics is impaired by false positives due to other gastrointestinal cancers such as colon cancer, gastric cancer, and liver cancer, as well as breast cancer and other gynecological cancers, lung cancer, and bronchial cancer. Benign diseases such as pancreatitis also result in false positive CA19-9 levels. CA19-9 sensitivity for pancreatic cancer diagnostics is further impaired by false negative results in patients that are negative for Lewis a/b antigen and will therefore not express CA19-9.

However, the level of CA 19-9 may be different between individuals, ranging from non-detectable levels (CA19-9 negative individuals) to patients having relatively high levels of CA 19-9, e.g. due to pre-existing conditions or genetic predispositions. Thus, the guidelines from the American Society of Clinical Oncology discourage the use of CA 19-9 as a general screening test for pancreatic cancer. The reason is that the test may be false negative in many cases, or abnormally elevated in people who have no cancer at all (false positive). The main use of CA 19-9 is therefore to see whether a pancreatic tumor is secreting it at a certain level. Thus, the sensitivity and reliability of existing tests using CA19-9 has to be improved, since the typical cut-off value of 37 U/ml of the current tests may not be suited for all patients.

As mentioned above, the CA19-9 assays currently available on the market do not take into account the individual patient set-up such as genetic predispositions or pre-existing conditions that may influence the expression of CA19-9. Thus, better assays that reduce the rates of false positive or false negative results and allow for a reliable diagnosis for each individual patient are highly needed.

Heart failure (HF) is commonly associated with the final common stage of many cardiovascular diseases and is defined as a clinical syndrome in which patients in the final stage show typical signs and symptoms of effort intolerance and/or fluid retention resulting from an abnormality of cardiac structure or function. Heart failure comprises a wide range of patients including patients with a systolic and/or diastolic dysfunction. Thus, heart failure may affect the right heart (pulmonary circulation), the left heart (body circulation) or both. Moreover, heart failure can be classified as "chronic" or "acute" according to the onset and duration of the disease. Chronic heart failure is a long-term condition, usually kept stable by the treatment of symptoms. In contrast, acute heart failure is related to a rapid onset of heart failure symptoms which can result in acute respiratory distress.

According to the ESC Guidelines for the diagnosis and treatment of acute and chronic heart failure, the plasma concentration of natriuretic peptides (NPs) can be used as an initial diagnostic test. The upper limit of normal in the non-acute setting for N-terminal pro-BNP (NT-proBNP) is 125 pg/mL; in the acute setting, higher values should be used (NT-proBNP < 300 pg/mL) (Ponikowski et al, 2016. ESC Guidelines for the diagnosis and treatment of acute and chronic heart failure: The Task Force for the diagnosis and treatment of acute and chronic heart failure of the European Society of Cardiology (ESC) Developed with the special contribution of the Heart Failure Association (HFA) of the ESC. European heart journal, 37(27), 2129-2200). However, as mentioned above, the currently available assays do not take into account the individual molecular and physiological set-up of a patient and do not consider potentially higher or lower personalized cutoff points.

Collerton et al. reported in the Newcastle 85+ Study that, at the suggested ESC cut off point of 125 pg/ml NT-proBNP, only a few cases of systolic dysfunction were missed (NPV 94-100%, depending on severity), but echocardiography (88%) and false positive rates (56-81 per 100 screened) were very high (Collerton et al, 2014. Utility of NT-proBNP as a rule-out test for left ventricular dysfunction in very old people with limiting dyspnoea: the Newcastle 85+ Study. BMC cardiovascular disorders, 14(1), 128). This study highlights the importance of further improving the currently used methods, e.g. by determining a personalized cutoff value for a biomarker such as NT-pro BNP for each patient.

Thus, there is a strong need to prevent false positive or false negative results in biomarker assays taking into account the molecular and physiological set-up of an individual patient, leading to a reliable diagnosis for each individual patient.

The technical problem underlying the present invention can be seen as the provision of means and methods for complying with the aforementioned needs. The technical problem is solved by the embodiments characterized in the claims and described herein below.

Therefore, the present invention relates to a method for determining a personalized cutoff value for a biomarker comprising the steps:

(a) determining in a sample of a subject the amounts of at least two metabolic analytes;
(b) providing predetermined constants which allow for transforming the determined amounts of the metabolic analytes into a cutoff value for the biomarker, wherein said predetermined constants being derived from discrimination between a diseased patient population and a control population; and
(c) determining a personalized cutoff value for a biomarker based on the amounts determined in step a) and said predetermined constants provided in step (b),

The method as referred to in accordance with the present invention includes a method which essentially consists of the aforementioned steps or a method which includes further steps. However, it is to be understood that the method, in a preferred embodiment, is a method carried out *ex vivo,* i.e. not practised on the human or animal body. The method, preferably, can be assisted by automation.

The term "biomarker" as used herein refers to a molecular species which serves as an indicator for a disease or disorder. Said molecular species typically is a molecule or several molecules present in a biological sample obtained from a patient, the concentration of which in said sample may be indicative of the presence of a disease or of a certain state of a disease or of the risk to develop a certain disease or prognostic for disease progression (e. g. relapse or metastases) or for treatment response. For example, biomarkers can be peptides, polypeptides, proteins, glycoproteins, hormones, cytokines or metabolites. Biomarkers may also be related to the presence of mutated genes or fusion genes and their protein products such as BRCA1 or *BCR-ABL1.* Preferably, the biomarkers according to the present invention are peptides, polypeptides or proteins including post-translationally modified variants such as truncated or glycosylated or phosphorylated proteins.

Biomarkers for different diseases and disorders are well known to those skilled in the art and include, for example, biomarkers for the diagnosis of cancer such as CA-125 (Cancer Antigen 125) for ovarian cancer, S100 for melanoma, PSA (Prostate Specific Antigen) for prostate cancer or CA19-9 (Carbohydrate-Antigen 19-9) for pancreatic cancer as well as biomarkers for the diagnosis of heart failure such as natriuretic peptides, including the atrial natriuretic peptide (ANP) or the brain natriuretic peptide (BNP) and parts thereof such as, preferably, brain natriuretic peptide (BNP) or N-terminal pro BNP (NT-proBNP), or biomarkers indicative for heart disease such as aortic stenosis or mitral insufficiency, namely troponins.

In a preferred embodiment of the method of the present invention the biomarker is NT-proBNP. The term "NT-proBNP" as used herein refers to the N-terminal brain natriuretic peptide or protein which consists of the N-terminal amino acid residues 1-76 remaining after BNP undergoes processing. Typically, BNP is synthesized as a 134-amino acid preprohormone (preproBNP), encoded by the human gene NPPB. Removal of the 25-residue N-terminal signal peptide generates the prohormone, proBNP, which is subsequently cleaved between arginine-102 and serine-103 by a specific convertase into NT-proBNP and the 32-amino acid polypeptide BNP-32. The 32-amino acid polypeptide BNP is typically secreted attached to the 76-amino acid N-terminal fragment in the prohormone called NT-proBNP. The nucleic acid and amino acid sequences of BNP and NT-proBNP are known to those skilled in the art and can be found in well-known databases such as NCBI gene and UniProtKB.

NT-proBNP is used as a biomarker for the diagnosis of heart failure. What is meant by the term "heart failure" is explained elsewhere in this specification. Several biomarker assays comprising NT-proBNP are currently available on the market. Typically, said biomarker assays have a cutoff value of 125 pg/ml for exclusion of heart failure, meaning that a patient is unlikely to suffer from heart failure if the value is below that cutoff. It is, however, known to those skilled in the art that said NT-proBNP assays may lead to false positive or false negative results and, thus, need to be further improved to allow a reliable diagnosis for each individual patient.

In a further preferred embodiment of the method of the present invention the biomarker is CA 19-9. The term "CA 19-9" refers to a tumor marker called carbohydrate antigen 19-9, also referred to as cancer antigen 19-9 or sialylated Lewis (a) antigen. CA 19-9 is used in the diagnosis of pancreatic cancer. The nucleic acid and amino acid sequences of CA 19-9 are known to those skilled in the art and can be found in well-known databases such as NCBI gene and UniProtKB.

It is known to those skilled in the art that CA 19-9 is used as a biomarker for the diagnosis of pancreatic cancer. What is meant by the term "pancreatic cancer" is explained elsewhere in this specification. Several biomarker assays comprising CA 19-9 are currently available on the market. Typically, said biomarker assays have a cutoff value of 37 U/ml, above or below which a patient is diagnosed as suffering from pancreatic cancer or not. It is, however, also known that said CA 19-9 assays may lead to false positive or false negative results and, thus, need to be further improved to allow a reliable diagnosis for each individual patient.

The term "metabolic analyte" as used herein refers to a molecular species, the presence or concentration of which in a sample is determined by analysis. Such molecular species within a cell can typically be assigned to metabolic pathways, in which one molecule is transformed through a series of steps into another molecule, e.g. by a sequence of enzymatic reactions. A metabolic analyte in accordance with the present invention, thus, encompasses all classes of molecular species including organic and inorganic compounds comprised in a sample or a biological material such as an organism. Preferably, the metabolic analyte is an organic compound.

Preferred metabolic analytes according to the present invention include amino acids such as alanine, cysteine, cystine, glutamate, isoleucine, phenylalanine, proline, taurine, or tyrosine, carbohydrates such as erythrol, glucosamine, glucose, glucose-1-phosphate, maltose or myo-Inositol, lipids or fatty acids such as sphingolipids, behenic acid, ceramides, cholesterylesters, diacylglycerols, eicoasenoic acid, glycerol-3-phosphate, isopalmitic acid, lauric acid, lignoceric acid, linoleic acid, lysophosphatidylcholines, oleic acid, palmitic acid, palmitoleic acid, phosphatidylcholines, sphingomyelins, triacylglycerols or tricosanoic acid, coenzymes Q9 or Q10, nucleobases such as hypoxanthine, pseudouridine, uric acid or uridine and vitamins such as beta-carotene, gamma-tocopherol or threonic acid.

Moreover, a metabolic analyte according to the present invention is not necessarily corresponding to a single molecular species. Rather, the metabolic analyte may comprise stereoisomers or enantiomeres of a compound. In addition, a metabolic analyte may also represent the sum of isomers of a biological class of isomeric molecules. Said isomers shall exhibit identical analytical characteristics in some cases and are, therefore, not distinguishable by various analytical methods including, for example, mass spectrometric analysis as described in more detail below. However, the isomers will share at least identical sum formula parameters and, thus, in the case of, e.g., lipids, an identical chain length and identical numbers of double bonds in the fatty acid and/or sphingo base moieties.

In the method according to the present invention, at least two metabolic analytes are to be determined as described elsewhere herein in more detail. Briefly, for quantitative determination, either the absolute or precise amount of the metabolic analytes will be determined or the relative amount of the metabolic analytes will be determined based on the value determined for the characteristic feature(s). However, more preferably, at least two metabolic analytes, most preferably at least three metabolic analytes, at least four metabolic analytes, at least five metabolic analytes or at least six metabolic analytes will be determined in order to strengthen specificity and/or sensitivity of the assessment.

In a preferred embodiment of the method of the present invention, wherein the biomarker is NT-pro-BNP, said at least two metabolic analytes are selected from the group consisting of the analytes shown in Table 1.

Table 1: List of preferred metabolic analytes to be used for determining a personalized cutoff for the biomarker NT-proBNP

| Metabolic analyte |
| --- |
| Phosphatidylcholine (PC) 16:0/18:2 |
| Sphingomyelin (SM) d18:1/23:1 |
| Sphingomyelin (SM) d18:2/23:0 |
| Sphingomyelin (SM) d17:1/24:1 |
| Triacylglyceride (TAG) 18:1/18:0/18:0 |
| Triacylglyceride (TAG) 16:0/16:0/18:1 |
| Cholesterylester (CE) 18:2 |

(continued)

| Sum of sphingomyelins |
|---|
| Sum of triacylglycerides |
| Sum of cholesterylesters |

More preferably, said at least two metabolic analytes in a preferred embodiment of the present invention, wherein the biomarker is NT-pro-BNP, are PC 16:0/18:2 and SM d18:1/23:1 or PC 16:0/18:2 and SM d18:2/23:0 or PC 16:0/18:2 and SM d17:1/24:1 or PC 16:0/18:2 and TAG 18:1/18:0/18:0 or SM d18:1/23:1 and TAG 18:1/18:0/18:0 or SM d18:2/23:0 and TAG 18:1/18:0/18:0 or SM d17:1/24:1 and TAG 18:1/18:0/18:0. Further preferred, said at least two metabolic analytes are the sum of two or more sphingomyelin species, in particular the sum of SM d18:1/23:1, SM d18:2/23:0 and SM d17:1/24:1 and the sum of two or more triacylglycerides species, in particular the sum of TAG 18:1/18:0/18:0 and TAG 16:0/16:0/18:1. Also preferably, the sum of sphingomyelins, the sum of triacylglycerides and/or the sum of cholesterylesters as determined by an enzymatic assay are determined.

As described elsewhere herein, a sample in accordance with the present invention may comprise a subfraction of metabolic analytes such as a subfraction of sphingomyelins obtained by any one of the techniques mentioned herein, which is representative for the amount of the total sphingomyelins. Similarly, subfractions of the total triacylglycerols, or the total cholesteryl esters, or the total lipoproteins may be used as samples. According to the present invention, the sample may also comprise a derivative derived from the sphingomyelins or the triacylglycerols which is representative for the amount of the total sphingomyelins or the total triacylglycerols. Also moreover, the sample according to the present invention may comprise a derivative derived from the total cholesteryl esters which is representative for the amount of said total cholesteryl esters. The total amount of sphingomyelins, triacylglycerides or cholesterylesters can also be determined by commercially available kits, for example the Sphiomgomyelin Quantification Colorimetric Assay Kit from BioVision incorporated, Milpitas (US) (Catalog # K600-100), the Cholesterol/Cholesteryl Ester Quantitation Colorimetric Kit II from BioVision, Inc., Milpitas (US) (Catalog # K623-100) or determination of the total amount of triacylglycerols and the total amount of cholesteryl esters plus cholesterol by a colorimetric enzyme assay using the cobas® 8000 system (Roche Diagnostics Limited, Rotkreuz, Switzerland) based on a method published by Wahlefeld et al. (Wahlefeld AW, Bergmeyer HU, eds. Methods of Enzymatic Analysis. 2nd English ed. New York, NY: Academic Press Inc 1974; 1831).

In a further preferred embodiment of the method of the present invention, wherein the biomarker is NT-proBNP, at least two metabolic analytes, most preferably at least three metabolic analytes, at least four metabolic analytes, at least five metabolic analytes or at least 6 metabolic analytes selected from the group of analytes listed in Table 1 will be determined. More preferably, said metabolic analytes to be determined in step a) of the method according to the present invention, wherein the biomarker is NT-pro-BNP, are PC 16:0/18:2 and TAG 18:1/18:0/18:0 and at least one sphingomyelin selected from the group consisting of SM d18:1/23:1, SM d18:2/23:0 and SM d17:1/24:1, or, more preferably the sum of the three sphingomyelins, SM d18:1/23:1, SM d18:2/23:0 and SM d17:1/24:1.

In a further preferred embodiment of the method of the present invention, wherein the biomarker is CA19-9, the at least two metabolic analytes are selected from the group consisting of the analytes shown in Table 2.

Table 2: List of preferred metabolic analytes to be used for determining a personalized cutoff for the biomarker CA 19-9

| Metabolic analyte |
|---|
| Proline |
| Tryptophan |
| Lysophosphatidylethanolamine (LPE) C18:2 |
| Sphingomyelin (SM) d18:2,C17:0 |
| Sphingomyelin (SM) d18:1,C17:0 |
| Ceramide (Cer) d18:2,C24:0 |
| Ceramide (Cer) d18:1,C24:0 |
| Sphingomyelin (SM) 41:2 |
| Sphingomyelin (SM) 35:1 |

(continued)

| Phosphatidylethanolamine C18:0,C22:6 |
| --- |
| Sphingomyelin (SM) d17:1,C16:0 |
| Histidine |

More preferably, said at least two metabolic analytes in a preferred embodiment of the present invention, wherein the biomarker is CA 19-9, are Proline and Tryptophane or Proline and LPE C18:2 or Proline and SM d18:2,C17:0 or Proline and SM d18:1,C17:0 or Proline and Cer d18:2,C24:0. Most preferably, said at least two metabolic analytes are Proline and LPE C18:2 or Proline and Cer d18:2,C24:0.

In a further preferred embodiment of the method of the present invention, wherein the biomarker is CA 19-9, more than two metabolic analytes, most preferably at least three metabolic analytes, at least four metabolic analytes, at least five metabolic analytes or at least 6 metabolic analytes selected from the group of analytes listed in Table 1 will be determined. Most preferably, said metabolic analytes to be determined in step a) of the method according to the present invention, wherein the biomarker is CA 19-9, are Proline and Lysophosphatidylethanolamine (LPE) C18:2 and Ceramide (Cer) d18:2,C24:0, or, Proline and Lysophosphatidylethanolamine (LPE) C18:2 and Ceramide (Cer) d18:2,C24 and Tryptophane, or, Proline and Lysophosphatidylethanolamine (LPE) C18:2 and Ceramide (Cer) d18:2,C24 and Tryptophane and Sphingomyelin (SM) d18:2,C17:0 and/or Sphingomyelin (SM) d18:1,C17:0.

The term "determining" or "determining the amount" as used herein refers to determining at least one characteristic feature of a compound. Said compounds include organic and inorganic compounds comprised by a sample of a subject, preferably organic compounds such as lipid metabolites or amino acids or proteins. According to the method of the present invention, at least one characteristic feature of each metabolic analyte of the at least two metabolic analytes are determined in a sample of a subject. Characteristic features in accordance with the present invention are features which characterize the physical and/or chemical properties including biochemical properties of each metabolic analyte. Such properties include, e.g., molecular weight, viscosity, density, electrical charge, spin, optical activity, colour, fluorescence, chemiluminescence, elementary composition, chemical structure, capability to react with other compounds, capability to elicit a response in a biological read out system (e.g., induction of a reporter gene or activation of a dye) and the like. Values for said properties may serve as characteristic features and can be determined by techniques well known in the art. Moreover, the characteristic feature may be any feature which is derived from the values of the physical and/or chemical properties of each metabolic analyte by standard operations, e.g., mathematical calculations such as multiplication, division or logarithmic calculus. Most preferably, the at least one characteristic feature allows the determination and/or chemical identification of each of said at least two metabolic analytes and its relative or absolute amount. Accordingly, the characteristic value, preferably, also comprises information relating to the abundance of each metabolic analyte from which the characteristic value is derived. For example, a characteristic value of a metabolic analyte may be a peak in a mass spectrum. Such a peak contains characteristic information of the metabolic analyte, i.e. the m/z information, as well as an intensity value being related to the abundance of the said metabolic analyte (i.e. its amount) in the sample.

Each metabolic analyte comprised by a sample may be, preferably, determined in accordance with the present invention quantitatively or semi-quantitatively. For quantitative determination, either the absolute or precise amount of the at least two metabolic analytes will be determined or the relative amount of the at least two metabolic analytes will be determined based on the value determined for each of the characteristic feature(s) referred to herein above. For example, a characteristic feature in form of an LC-MS/MS peak area ratio relative to an internal standard, or the absolute concentration of a metabolic analyte based on an external calibration standard may be determined. The relative amount may be determined in a case were the precise amount of a metabolic analyte can or shall not be determined. Preferably, the amounts of the at least two metabolic analytes are determined in form of LC-MS/MS peak area ratios or ng/mL or $\mu$mol/L. Most preferably, the amounts of the at least two metabolic analytes are determined in form of ng/mL.

Suitable internal and external standards to be used in accordance with the present invention are known to those skilled in the art and include, for example, internal standard solutions containing phosphatidylcholine (PC) 19:0/19:0 (e.g. a solution containing PC 19:0/19:0 in an amount of 41,28 $\mu$g/ml available from Avanti Polar Lipids, CA, US) or commercially available external standard solutions for phosphatidylcholine, sphingomyelin, cholesterylester, and triglyceride species.

The term "at least the amounts", preferably means that, in principle, further metabolic analytes could be determined. Thus, according to the present invention, the amounts of at least two metabolic analytes, more preferably at least three, at least four, at least five or at least six metabolic analytes are determined in step a) of the method of the present invention.

It is also envisaged that more than one metabolic analyte belonging to the same analyte class, e.g. the class of sphingomyelins or the class of triacylglycerides is determined. In a preferred embodiment, the determination of the amounts of the at least two metabolic analytes comprises the determination of the sum of more than one or all sphingomyelins and the sum of more than one or all triacylglycerides that may be detected in a sample of a subject as explained elsewhere herein.

Moreover, determining the amounts of at least two metabolic analytes in step a) according to the method of the present invention, preferably includes using a compound separation step prior to the analysis. Preferably, said compound separation step yields a time-resolved separation of the at least two metabolic analytes comprised by the sample. Suitable techniques for separation to be used preferably in accordance with the present invention include chromatographic separation techniques such as liquid chromatography (LC), high performance liquid chromatography (HPLC), gas chromatography (GC), thin layer chromatography, size exclusion or affinity chromatography. These techniques are known to those skilled in the art and can be applied without further ado. Preferably, LC and/or GC techniques shall be used.

Means and methods for the determination of metabolic analytes are well known in the art. Preferably, mass spectrometry is used, in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). More preferably, LC-MS and/or GC-MS are used as described in more detail below. The aforementioned techniques and suitable devices are known in the art and disclosed, for example, in US 4,540,884 or US 5,397,894, or Niessen et al, 1995 (Niessen et al, 1995, Liquid chromatography-mass spectrometry general principles and instrumentation. Journal of Chromatography A, 703(1-2), 37-57), the disclosure content of which is hereby incorporated by reference.

As an alternative or in addition to mass spectrometry techniques, the following techniques may be used for the determination of said at least two metabolic analytes: nuclear magnetic resonance (NMR), magnetic resonance imaging (MRI), Fourier transform infrared analysis (FT-IR), ultraviolet (UV) spectroscopy, refraction index (RI), photometric detection, fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing, comparison or evaluation, is, preferably, assisted by suitable computer programs and databases. Automation as described herein allows using the method of the present invention in high-throughput approaches.

Furthermore, the at least two metabolic analytes can also be determined by a specific chemical or biological assay. Said assay shall comprise means which allow to specifically detect one or more of the at least two metabolic analytes in the sample. Preferably, said means are capable of specifically recognizing the chemical structure of a metabolic analyte, e.g. an enzyme or an antibody or another protein that interacts with the metabolic analyte, or are capable of specifically identifying a metabolic analyte based on its capability to react with other compounds or its capability to elicit a response in a biological read out system (e.g. the induction of a reporter gene). Preferably, the amounts of the at least two metabolic analytes are determined by an enzymatic assay as explained elsewhere herein in more detail. Antibodies as referred to herein include both polyclonal and monoclonal antibodies, as well as fragments thereof, such as Fv, Fab and F(ab)2 fragments that are capable of binding the antigen or hapten. It is well known that specific antibodies for a specific metabolic analyte may be obtained by using said metabolic analyte as antigen by methods known to those skilled in the art. Thus, the present invention also includes humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins that can specifically recognize a metabolic analyte are, preferably, enzymes which are involved in the metabolic conversion of said metabolic analyte. It is well known that metabolites can be converted by specific enzymes in several consecutive reactions, resulting in activation of a dye that can be determined photometrically. As an example, sphingomyelins can be converted by enzymes such as sphingomyelinase into ceramide and phosphocholine, while phosphocholine can be converted by enzymes such as alkaline phosphatase into phosphate and choline, while choline can be converted by enzymes such as choline esterase into betaine aldehyde and $H_2O_2$, while $H_2O_2$ can be converted by enzymes such as peroxidases into $H_2O$, thereby activating a dye that can be detected photometrically and can be used for quantification of the amount of sphingomyelin in a sample by comparison with a standard curve. Moreover, antibodies may be used as a basis to generate oligopeptides which specifically recognize a metabolic analyte. These oligopeptides shall, for example, comprise the enzyme's binding domains or pockets for the said metabolic analyte. Suitable antibody and/or enzyme based assays include, for example colorimetric assay, RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the at least two metabolic analytes may also be determined based on their capability to react with other compounds, i.e. by a specific chemical reaction. Further, the metabolic analyte may be determined in a sample due to its capability to elicit a response in a biological read out system. The biological response shall be detected as read out indicating the presence and/or the amount of the metabolic biomarker comprised by the sample. The biological response may be, e.g., the induction of gene expression or a phenotypic response of a cell or an organism.

Moreover, the metabolic analytes and in particular, the aforementioned total amounts of triacylglycerols or sphingomyelins or cholesteryl esters can be determined by any suitable detection agent or detection device which allow for a specific determination of the total amounts. Suitable detection agents, preferably, include agents which specifically bind to the at least one cholesterol parameter (e.g. cholesteryl esters), the total sphingomyelins or the total triacylglycerides. Such agents may be antibodies or aptamers whose binding to cholesteryl esters, triacylglycerols or sphingomyelins can be determined by means well known in the art, such as secondary or higher order antibodies linked to a detectable label such as a fluorophore or chromophore. Suitable measurement methods also include RIA (radioimmunoassay), ELISA (enzyme-linked immunosorbent assay), sandwich enzyme immune tests, electrochemiluminescence immunoassays such as electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests or affimer technology based tests (Avacta, US). Further methods known in the art, such as gel electrophoresis, 2D gel electrophoresis, SDS polyacrylamid gel electrophoresis (SDS-PAGE), Western Blotting, surface plasmon resonance, IR spectroscopy, and mass spectrometry. The total amounts of triacylglycerols or sphingomyelins or cholesteryl esters can, however, also be determined by detection devices which allow for the specific detection of the said biomarkers.

Preferably, the amount of a metabolic analyte and in particular the total amount of cholesteryl esters, of triacylglycerols and/or of sphingomyelins are determined enzymatically. To this end, it is envisaged that enzymes are applied which specifically recognize cholesterylesters, triacylglycerols or sphingomyelins as substrates and which convert said substrates such that a detectable signal can be generated. Typically, the enzymatic conversion generates, e.g., redox equivalents e.g. in the form of $H_2O_2$. Said redox equivalents can be in turn detected in a further reaction by a peroxidase (such as a horseradish peroxidase) and a chromogenic substrate. The substrate is typically oxidized by the peroxidase using $H_2O_2$ as the oxidizing agent. The catalyzed reaction in the presence of $H_2O_2$, peroxidase, and the substrate typically results in a characteristic change that is detectable by spectrophotometric methods. E.g., the peroxidase catalyzes the conversion of chromogenic substrates into colored products, or produces light when acting on chemiluminescent substrates. For example, DAOS (N-ethyl-N-(2-hydroxy-3-sulfopropyl)-3,5-dimethoxyaniline) plus 4-aminoantipyrine in the presence of $H_2O_2$ and peroxidase results in the oxidative coupling of DAOS and 4-aminoantipyrine to form a blue chromogen. This chromogen can be e.g. detected by measuring the absorbance of light at about 590 nm. Alternatively, 10-acetyl-3,7-dihydroxyphenoxazine can be used as substrate for peroxidase that enables detection of $H_2O_2$. This non-fluorescent reagent reacts with $H_2O_2$ to produce resorufin, a fluorescent compound. In another alternative 4-aminophenazon plus 4-chlorphenol in the presence of $H_2O_2$ and peroxidase result in the formation of 4-(p-benzochinon-monoimino)-phenazon, a red colored product, which can be determined by photometric means.

The total amount of sphingomyelins, triacylglycerides or cholesterylesters can also be determined by commercially available kits, for example the Sphiomgomyelin Quantification Colorimetric Assay Kit from Bio Vision incorporated, Milpitas (US) (Catalog # K600-100), the Cholesterol/Cholesteryl Ester Quantitation Colorimetric Kit II from BioVision, Inc., Milpitas (US) (Catalog # K623-100) or determination of the total amount of triacylglycerols and the total amount of cholesteryl esters plus cholesterol by a colorimetric enzyme assay using the cobas® 8000 system (Roche Diagnostics Limited, Rotkreuz, Switzerland) based on a method published by Wahlefeld et al. (Wahlefeld AW, Bergmeyer HU, eds. Methods of Enzymatic Analysis. 2nd English ed. New York, NY: Academic Press Inc 1974; 1831).

In a preferred embodiment, the enzymes used for the enzymatic determination of the amount(s) of at least one cholesterol parameter, total triacylglycerols and/or total sphingomyelins are non-human enzymes. Preferably, the enzymatic determination of total triacylglycerols and/or total sphingomyelins and/or total cholesterylesters is based on artificial means, in particular on combinations which do not naturally occur in the sample to be tested.

For the enzymatic determination, sample pre-treatment may be required in order to allow the enzymes to get access to the metabolic analytes in a suitable way. Typically, the samples are physically and/or chemically treated such that a metabolic analyte, for example sphingomyelins, triacylglycerols or cholesteryl esters, are released into solution. Physical treatments may include the application of heat or physical homogenization process. Chemical treatments may include solubilisation and extraction treatments by suitable buffers and agents, such as detergents, wetting agents and organic, preferably, apolar solvents. The metabolic analyte obtained, i.e. the total sphingomyelins or the total cholesteryl esters may than be diluted in an aqueous buffer system which allows for optimal enzyme activity. Such buffers are usually pH- and salt- optimized for the respective detection enzymes.

As described above, said determining of the at least two metabolic analytes, preferably, comprises mass spectrometry (MS). Mass spectrometry as used herein encompasses all techniques which allow for the determination of the molecular weight (i.e. the mass) or a mass variable corresponding to a compound, i.e. a metabolic analyte. More preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: 1) selection of a mass/charge quotient (m/z) of an ion created by ionisation in a first analytical quadrupole of the mass spectrometer, 2) fragmentation of the ion selected in 1) by applying an acceleration voltage in an additional subsequent quadrupole which is filled with a collision gas and acts as a collision chamber, 3) selection of a mass/charge quotient of an ion created by the fragmentation process in 2) in an additional subsequent quadrupole, whereby 1) to 3) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in WO 03/073464.

More preferably, said mass spectrometry is liquid chromatography (LC) MS and/or gas chromatography (GC) MS. Liquid chromatography as used herein refers to all techniques which allow for separation of compounds (e.g. of the at least two metabolic analytes to be determined in accordance with the present invention) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as referred to in accordance with the present invention, typically, operates comparable to liquid chromatography. However, rather than having the compounds (i.e. the metabolic analytes) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography, it is preferably envisaged that the compounds are derivatised prior to gas chromatography. Suitable techniques for derivatisation are well known in the art and include, for example, methoxymation and trimethylsilylation of polar compounds and transmethylation, methoxymation and trimethylsilylation of non-polar compounds.

The term "sample" as used herein refers to samples from body fluids, preferably, blood, plasma, serum, saliva, urine or feces, or samples derived, e.g., by biopsy, from cells, tissues or organs. Preferably, the sample is a blood, serum, plasma, urine, salvia, feces or tissue sample. More preferably, the sample is a blood, serum or plasma sample, most preferably, a plasma sample. In a yet preferred embodiment of the present invention said sample is a blood, plasma or serum sample derived from a subject suffering from or suspected to suffer from or at risk of heart failure or a sample derived from the heart of said subject. In another preferred embodiment of the present invention said sample is a blood, serum, plasma, urine, salvia, feces or tissue sample, preferably a cancer tissue, sample derived from a subject suffering from or suspected to suffer from or at risk of cancer, in particular pancreatic cancer. Biological samples can be derived from a subject as specified elsewhere herein. Techniques for obtaining the aforementioned different types of biological samples are well known in the art. For example, blood samples may be obtained by blood taking while tissue or organ samples are to be obtained, e.g., by biopsy.

The aforementioned samples are, preferably, pre-treated before they are used for the method of the present invention. Said pre-treatment may include treatments required to release or separate the compounds, i.e. said at least two metabolic analytes, or to remove excessive material or waste. Suitable pre-treatment techniques are known to those skilled in the art and include centrifugation, extraction, fractioning, ultrafiltration, protein precipitation followed by filtration and purification and/or enrichment of compounds. Moreover, other pre-treatments may be carried out in order to provide the compounds in a form or concentration suitable for analysis. For example, if gas-chromatography coupled mass spectrometry is used in the method of the present invention, it will be necessary to derivatize the compounds prior to the said gas chromatography. Suitable and necessary pre-treatments depend on the means used for carrying out the method of the invention and are well known to the person skilled in the art. Pre-treated samples are thus also comprised by the term "sample" as used in accordance with the present invention. A sample or pre-treated sample may also comprise a subfraction of metabolic analytes such as a subfraction of sphingomyelins obtained by any one of the techniques mentioned herein, which is representative for the amount of the total sphingomyelins. Similarly, subfractions of the total triacylglycerols or the total cholesteryl esters may be used. It might be necessary to release the said metabolites from transport lipoproteins of the blood, e.g., apo-lipoproteins which form, e.g., high density, low density or very low density lipoproteins (HDL, LDL or VLDL). Moreover, the sample may be enriched for HDL, LDL and/or VLDL by, e.g., centrifugation and/or fractioning.

It will be understood that the determination of the amounts of the at least two metabolic analytes in a sample of a subject according to the present invention, preferably includes a separation step, i.e. a step in which the metabolic analytes or compounds such as amino acids, lipids, fatty acids or vitamins, comprised by the sample are separated. Preferably, the separation of the compounds is carried out by chromatography, in particular by liquid chromatography (LC) or high performance liquid chromatography (HPLC). Preferably, the pre-treatment of the sample should allow for a subsequent separation of the metabolic analytes, in particular the at least two metabolic analytes as referred to above, comprised by the sample. Molecules of interest, in particular the metabolic analytes as referred to above, may be extracted in an extraction step which comprises mixing of the sample with a suitable extraction solvent. Suitable extraction solvents are known to those skilled in the art and include, for example, dichloromethane (DCM), chloroform, tertiary butyl methyl ether, ethyl ethanoate, isooctane, methanol, ethanol, isopropanol and dimethyl sulfoxide (DMSO). The extraction solvent shall be capable of precipitating the e.g. proteins, in a sample, thereby facilitating the, preferably, centrifugation-based, removal of contaminants which otherwise would interfere with the subsequent analysis of the metabolic analytes as referred above.

The sample shall be derived from a subject as described below. Said subject may be a fasted or non-fasted subject. Preferably, said subject is a fasted subject who refrained from food and beverages, except for water, prior to obtaining the sample to be tested. Preferably, a fasted subject refrained from food and beverages, except for water, for at least eight hours prior to obtaining the sample to be tested. More preferably, the sample has been obtained from the subject after an overnight fast. Thus, the sample derived from the subject can be a fasted sample, preferably a fasted blood, serum or plasma sample.

The term "subject" as used herein relates to animals and, preferably, to mammals. More preferably, the subject is a primate and, most preferably, a human. In a preferred embodiment of the present invention the subject is suffering from heart failure or is at risk to suffer from or suspected to suffer from heart failure. In a further preferred embodiment of the present invention the subject is suffering from pancreatic cancer or is at risk to suffer from or suspected to suffer from pancreatic cancer. More preferably, such a subject being at increased risk has one or more relatives suffering from heart failure or pancreatic cancer and/or has a defined genetic predisposition for developing heart failure or pancreatic cancer. Symptoms and characteristics of the aforementioned diseases and disorders are well known in the art and also referred to elsewhere in this specification. Preferably, the subject, however, is besides the aforementioned diseases and disorders apparently healthy.

The term "predetermined constants" relates to predetermined values based on a predefined dataset that comprises data from a diseased patient population and a control population. Preferably said predefined dataset consists of biomarker values, preferably in absolute amounts such as pg/ml, as well as metabolic analyte values, preferably in absolute amounts such as ng/ml, derived from a diseased patient population and a control population. In a preferred embodiment of the present invention, said predefined dataset consists of NT-proBNP biomarker values as well as metabolic analyte values from a diseased patient population and controls, preferably, wherein patients in said diseased patient population suffer from heart failure, more preferably from HFrEF, and wherein said metabolic analyte values preferably comprise the amounts of at least two of the metabolic analytes listed in Table 1. In yet a preferred embodiment of the present invention, said predefined dataset consists of CA19-9 biomarker values as well as metabolic analyte values from a diseased patient population and controls, preferably, wherein patients in said diseased patient population suffer from cancer, preferably from pancreatic cancer, and wherein said metabolic analyte values preferably comprise the amounts of at least two of the metabolic analytes listed in Table 2.

Said predefined dataset shall allow for discrimination between a diseased patient population and a control population not suffering from the respective disease and shall provide predetermined constants that, together with the amounts of at least two metabolic analytes determined in step a) of the method of the present invention, allow for determining a personalized cutoff value for the biomarker. Mathematical procedures that allow for said discrimination are well known in the art and described in more detail elsewhere herein. Preferably, those procedures or said discrimination comprises linear regression analysis.

Preferable, the predefined dataset has a predefined size, i.e. a predefined number of datapoints that allow for a statistically significant result. The size of the dataset that is needed to derive a statistically significant result may vary, e.g. depending on the number and scattering of the data points within a dataset. It is known to those skilled in the art that the power of a binary hypothesis test is the probability that the test correctly rejects the null hypothesis when a specific alternative hypothesis is true. Thus, the statistical power typically ranges from 0 to 1, and as statistical power increases, the probability of making a type II error (wrongly failing to reject the null) decreases. Preferably, a result with a power of at least 70 percent (0.7; leaving a 30 percent chance of a type II error), at least 80 percent (0.8; leaving a 20 percent chance of a type II error) or at least 90 percent (0.9; leaving a 10 percent chance of a type II error) shall be achieved. Most preferably, the predefined dataset derived from a diseased patient population and a control population allows for calculating a statistically significant result with a power of at least 80 percent.

Preferably, the predetermined constants as referred to herein comprise scaling factors specific for a metabolic analyte, i.e. scaling factors specific for each of said at least two metabolic analytes to be determined in step (a) of the method according to the present invention. More preferably, the scaling factors that are derived from the predefined dataset are specific to a metabolic analyte that may be disregulated in a disease, preferably a metabolic analyte as shown in Table 1 and/or 2. Preferably, the scaling factors denote the mean (also referred to herein as scaling factor mi) and the standard deviation (also referred to herein as scaling factor si) of the log of the metabolic analyte to be scaled. Moreover, the predetermined constants preferably encompass weigthing factors specific for a metabolic analyte (also referred to herein as weighting factor or coefficient wi), i.e. weigthing factors specific for each of said at least two metabolic analytes to be determined in step (a) of the method according to the present invention. More preferably, the weighting factors that are derived from the predefined dataset are specific to a metabolic analyte that may be dysregulated in a disease, preferably a metabolic analyte as shown in Table 1 and/or 2, and denote the weighting of the analyte in the overall result, i.e. the proportion that the analyte contributes to the overall result (the personalized cutoff value). Preferably, said weighting factor or coefficient (wi) is derived from the predefined dataset by linear regression.

Furthermore, the predetermined constants preferably comprise values related to the biomarker that are derived from the predefined dataset, i.e. from the discrimination between a diseased patient population and a control population as explained elsewhere herein. More preferably, three predetermined values related to the biomarker are used in the method according to the present invention, also referred to herein as constants c1, c2 and c3. Constant c1 as used herein denotes the mean value of the log transformed biomarker values, e.g. log-NT-proBNP values, of the predefined data set. Constant c2 denotes the quotient of the standard deviation of the log transformed biomarker values, e.g. log-NT-proBNP values, and the biomarker coefficient, e.g. NT-proBNP coefficient, of the predefined data set. Constant c3 (also called logit, Prediction Propability Cutoff - w0) denotes the difference of the log of the Prediction Propability Cutoff and the coefficient w0 of the predefined data set. While c1 and c2 are dependent on the biomarker values, c3 is dependent on all values in the predefined dataset. In particular, c1 is dependent on the biomarker values, e.g. NT-proBNP values, in the predefined dataset, e.g. the absolute amount of the biomarker (e.g. NT-proBNP) in diseased and control subjects. C2 is dependent on the the biomarker values, e.g. NT-proBNP values, in the predefined dataset and the weigthing of the biomarker (NT-proBNP) in the predefined dataset. C3 is dependent on all values in the predefined dataset, e.g. the values for the biomarker NT proBNP and values for the metabolic analytes such as the absolute amounts of TAG 18:1/18:0/18:0, SM d18:1/23:1, SM d18:2/23:0, SM d17:1/24:1 and PC 16:0/18:2.

Providing predetermined constants according to step b) of the method of the present invention preferably encompasses deriving values for the scaling factors mi and si, the weighting factor or coefficients wi and the constants c1, c2, c3 from a predefined dataset, preferably comprising data from a diseased patient population such as values for metabolic analytes referred to in Table 1 and the biomarker NT-proBNP in patients suffering from HErEF, and values from a control population, e.g. values for metabolic analytes referred to in Table 1 and the biomarker NT pro BNP from control subjects.

In a preferred setting, the scaling factors mi and si are adjusted rather than the coefficients wi, because it allows the logistic regression model with the coefficients wi to remain independent of the analytical method used. The logistic regression model or the mathematical formula, repectively, that is preferably used to determine a personalized cutoff value for a biomarker based on the amounts of said at least two metabolic analytes determined in step a) of the method of the present invention and said predetermined constants provided in step (b) of the method of the present invention, derived from discrimination between a diseased patient population and a control population, is shown below. The formula shown below refers to the biomarker NT-proBNP and metabolic analytes determined in a diseased patient population suffering from HFrEF and controls, providing the predetermined constants mi, si, wi, c1, c2 and c3. In a preferred setting, the result of the calculation, i.e. the linear regression model, is displayed as log-transformed result (10^x).

$$x1 = m1 + \frac{s1}{w1} * \left(logit(p) - w0 - \sum_{i=1}^{n} wi \frac{zi - mi}{si}\right)$$

$$x(NTproBNP)$$
$$= m(NTproBNP) + \frac{s(NTproBNP)}{w(NTproBNP)}$$
$$* \left(logit(Prediction\ Propability\ Cutoff) - w0 - \sum_{i=1}^{n} wi \frac{zi - mi}{si}\right)$$

$$x = c1 + c2 * (c3 - \sum_{i=1}^{n} wi \frac{zi - mi}{si})$$

| Factor | Description | Dependent on |
|---|---|---|
| x | Variable to be determined | All factors |
| zi | Value of the metabolic analyte | Amount, preferably absolute amount, of the metabolic analyte to be determined in step a) of the method of the present invention |
| mi | Scaling factor for metabolic analyte | Metabolic analyte value (preferably absolute amount) in the predefined dataset |
| si | Scaling factor for metabolic analyte | Metabolic analyte value (preferably absolute amount) in the predefined dataset |
| wi | Weighting factor for metabolic analyte | Metabolic analyte value in the predefined dataset |
| i | Metabolic analyte index | Specific metabolic analyte |
| c1 | Constant of the model | NT-proBNP values in the predefined data set |
| c2 | Constant of the model | NT-proBNP values and weighting of NT-proBNP in the predefined data set |
| c3 | Constant of the model | All values of the predefined data set |

It is known to those skilled in the art that the here disclosed linear regression model may be based on any suited predefined dataset, e.g. a predefined data set derived from a pancreatic cancer patient population and a control population suffering from chronic pancreatitis and/or a control population without pancreatic disease, that allows for determining a personalized cutoff value for CA19-9, as explained elsewhere herein.

The term "diseased patient population" refers to a population of patients that have a predefined disease. Having a predefined disease means that all patients in the population suffer from a certain disease or disorder, or a certain group of diseases or disorders. It is well known to the skilled artisan how to determine whether a subject or patient is suffering from a certain disease, for example by determining symptoms associated with said disease and/or determining the presence or amount of one or more biomarker(s) associated with a disease. Symptoms accompanying certain diseases, for example heart failure or pancreatic cancer, are well known from standard text books of medicine such as Stedmen or Pschyrembl. Preferably, said disease of the patient population is indicated by a biomarker, more preferably by the biomarker NT-proBNP or by the biomarker CA19-9 as described elsewhere herein. In a preferred embodiment of the present invention, said diseased patient population suffers from heart failure, more preferably from HFrEF. In a further preferred embodiment of the present invention, said diseased patient population suffers from pancreatic cancer.

The term "control population" refers to a population of subjects that do not have a predefined disease such as heart failure or cancer. Controls might be healthy or might have a definite disease different from the predefined disease dependent on the use case, such as chronic pancreatitis compared to pancreatic cancer Cases. Cases as well as Controls might have additional comorbidities. Said controls shall preferably not exhibit any of the symptoms, clinical signs or parameters of the predefined disease. Preferably, said predefined disease is indicated by a biomarker and said control subject or said subjects within the control population do not show a significant dysregulation of said biomarker. More preferably, said biomarker is NT-proBNP or CA19-9 as described elsewhere herein.

The term "heart failure" as used herein relates to an impaired function of the heart. It is a progressive disorder in which the heart fails to pump oxygenated blood at a rate sufficient to meet the metabolic needs of the tissues. Preferably, the term heart failure as used herein relates to congestive heart failure (CHF). Heart failure is known in the art as the final common stage of many cardiovascular diseases and is typically defined as a clinical syndrome in which patients in the final stage show typical signs and symptoms of effort intolerance and/or fluid retention resulting from an abnormality of cardiac structure or function. As outlined further herein below, heart failure in the context of the present invention encompasses both symptomatic forms and asymptomatic forms of heart failure. Accordingly, asymptomatic left ventricular systolic dysfunction or asymptomatic diastolic dysfunction may be diagnosed. Typical symptoms of heart failure include dyspnea, chest pain, dizziness, confusion, pulmonary and/or peripheral edema. Two subsets of heart failure with different pathophysiology are typically described based on a measurement of left ventricular ejection fraction (LVEF), which is the percentage of the total amount of blood in the left ventricle that is pushed out with each heartbeat: Heart failure with reduced left ventricular ejection fraction (HFrEF) and heart failure with preserved left ventricular ejection fraction (HFpEF). Preferably, heart failure as used herein relates to systolic heart failure or heart failure with reduced ejection fraction (HFrEF).It will be understood that the occurrence of the symptoms as well as their severity may depend on the severity of heart failure and the characteristics and causes of the heart failure, systolic or diastolic or restrictive i.e. right or left heart located heart failure. Further symptoms of heart failure are well known in the art and are described in the standard text books of medicine, such as Stedman or Brunnwald.

As used herein, the term "pancreatic cancer" or "pancreas cancer" relates to a cancer which is derived from pancreatic cells. Preferably, pancreatic cancer as used herein is pancreatic adenocarcinoma or pancreatic ductal adenocarcinoma (PDAC). Preferably, the pancreatic cancer is a resectable pancreatic cancer, i.e., preferably, is a pancreatic cancer at a tumor stage permitting, preferably complete, resection of the tumor from the subject. More preferably, said pancreatic cancer is a pancreatic cancer of tumor stage IA-IIB. The symptoms accompanying pancreatic cancer are well known from standard text books of medicine such as Stedmen or Pschyrembl and include severe abdominal pain, lower back pain, and in some cases jaundice.

The term "personalized cutoff value" for a biomarker, also referred to as "individual biomarker cutoff' or "personalized biomarker cutoff' refers to a value that defines a threshold level of said biomarker for an individual person. The personalized cutoff value is based on the amounts of the at least two metabolic analytes to be determined in step a) and the predetermined constants provided in step b) of the method according to the present invention. Said personalized biomarker cutoff can be applied for the diagnosis of a certain disease, e.g. of heart failure using a personalized biomarker cutoff for NT-proBNP or of pancreatic cancer using a personalized biomarker cutoff for CA19-9. Said personalized biomarker cutoff preferably reduces the number of false-positive or false-negative results in comparison to using biomarker assays currently available at the market with fixed, not individual, cutoff values, for example, NT-proBNP (N-terminal pro-Brain Natriuretic Peptide) biomarker assays with a typical cutoff of 125 pg/ml for the exclusion of heart failure and CA 19-9 (cancer antigen 19-9) biomarker assays with a typical cutoff of 37 U/ml for the diagnosis of pancreatic cancer. The personalized biomarker cutoff (taking into account the metabolic state of the patient, e.g. the values of at least two metabolic analytes to be determined in step a) of the method of the present invention) may, thus, be higher, lower or, in rare cases, equal to the typical cutoff of the currently used assays, e.g. higher or lower than 125 pg/ml NT-proBNP or higher or lower than 37 U/ml CA19-9, allowing for a better diagnosis of the individual patient.

In a preferred embodiment of the method of the present invention, the determined personalized cutoff value for the biomarker, wherein the biomarker is NT-proBNP, is lower, higher or equal to 125 pg/ml. Preferably, the personalized cutoff value for the biomarker, wherein the biomarker is NT-proBNP, is in the range of about 5 pg/ml to about 5000 pg/ml. Preferably, said personalized cutoff value is in the range of about 50 pg/ml to about 1000 pg/ml.

In a further preferred embodiment of the method of the present invention, the determined personalized cutoff value for the biomarker, wherein the biomarker is CA 19-9, said personalized cutoff value is lower, higher or equal to 37 Units/ml. Preferably said personalized cutoff value for the biomarker, wherein the biomarker is CA 19-9, is in the range of about 0,1 Units/ml to about 1600 Units/ml. More preferably, said predetermined threshold is in the range of about 2 Units/ml to about 1000 Units/ml, about 2 Units/ml to about 500 Units/ml, about 2 Units/ml to about 200 Units/ml, about 5 Units/ml to about 200 Units/ml, about 5 Units/ml to about 100 Units/ml, about 10 Units/ml to about 200 Units/ml, about 10 Units/ml to about 100 Units/ml, about 20 Units/ml to about 50 Units/ml or about 30 Units/ml to about 50 Units/ml or about 30 Units/ml to about 40 Units/ml. More preferably, said predetermined threshold is about 2 Units/ml, about 5 Units/ml, about 10 Units/ml, about 20 Units/ml, about 30 Units/ml, about 35 Units/ml, about 37 Units/ml, about 40 Units/ml, about 50 Units/ml, about 60 Units/ml, about 80 Units/ml, about 100 Units/ml, about 150 Units/ml, about 200 Units/ml.

About as referred to herein refers to any specific value referred to in this specification, e.g. a personalized cutoff value of NT-proBNP of about 1000 pg/ml, including any variation which is within the range of +/-20%, +/-10%, +/-5%, +/-4%, +/-3%, +/-2% or +/-1%.

The personalized cutoff can be determined by applying mathematical methods or calculations known to those skilled in the art, e.g. by multiplication, division, logarithmic calculation and the like. Preferably, the determination of the personalized cutoff is based on the amounts of said at least two metabolic analytes referred to above. The algorithm used for calculation is, preferably, derived from statistical analyses as also explained above. Methods for determining a suitable algorithm are well known in the art and include Significance Analysis of Microarrays, Tree Harvesting, CART, MARS, Self-Organizing Maps, Frequent Item Set, Bayesian networks, Prediction Analysis of Microarray (PAM), SMO, Simple Logistic Regression, Logistic Regression, Multilayer Perceptron, Bayes Net, Naive Bayes, Naive Bayes Simple, Naive Bayes Up, IB1, Ibk, Kstar, LWL, AdaBoost, ClassViaRegression, Decorate, Multiclass Classifier, Random Committee, j48, LMT, NBTree, Part, Random Forest, Ordinal Classifier, Sparse Linear Programming (SPLP), Sparse Logistic Regression (SPLR), Elastic net, Support Vector Machine, Prediction of Residual Error Sum of Squares (PRESS), Penalized Logistic Regression, Mutual Information. Preferably, the personalized cutoff algorithm is determined with or without correction for confounders, e.g. age, sex, BMI or others.

Said personalized biomarker cutoff is, preferably, determined via a linear regression model where the amounts of the at least two metabolic analytes (i.e. the absolute amounts in form of ng/ml or the relative amounts in form of peak area ratios) are used as input data for the metabolic features. More preferably, said logistic regression method comprises elastic net regularization. Values of different dimensions or units for the metabolites may be used as the values will be mathematically transformed for calculation of the personalized cutoff. Accordingly, values for absolute concentrations may be combined e.g. with absorption values. The metabolites are, preferably, weighted in accordance with their significance for the establishment of the diagnosis. Based on the combination of metabolites and the patient population, the weight of a specific metabolite, as indicated by said predermined constants decribed in more detail elsewhere herein, may be different.

Said personalized biomarker cutoff can be used to determine whether a subject suffers from a disease, e.g. from heart failure in case of the biomarker NT-proBNP or from pancreatic cancer in case of the biomarker CA19-9. Application of said personalized biomarker cutoff shall decrease the number of false-positive or false-negative results in biomarker assays for the diagnosis of a certain disease, preferably of heart failure or pancreatic cancer as explained above. Further information on personalized biomarker thresholds or biomarker cutoff values can also be found in the accompanying Examples .

Preferably, said determining a personalized cutoff value for a biomarker in step c) comprises calculating the said cutoff according to formula I:

$$x = c1 + c2 * (c3 - \sum_{i=1}^{n} wi \frac{zi - mi}{si})$$

Where x is the log10 transformed personalized cutoff, while zi are the log-transformed metabolite analytical results (preferably, taking into account the precise amounts of the at least two metabolic analytes in ng/ml), mi and si are feature-specific scaling factors, and wi are the coefficients of the model and n is the total number of metabolites. c1, c2 and c3 are predetermined constants, i.e. constants from the model, where c1 and c2 are derived from biomarker features of the respective predefinded dataset as described elsewhere herein, i.e. a dataset derived from a diseased patient population and a control population, while c3 is derived from the logistic regression. The scaling factors, coefficients and constants for the personalized cutoff of a biomarker are determined by the model based on said predefined dataset with values for the metabolites and for the biomarker from samples of subjects classified as cases or controls as specified in detail elsewehere herin and in the accompanying examples.

It is well known in the art that a biomarker cannot be reasonably used in samples of subjects known or assumed to be negative for that respective biomarker, e.g. CA19-9 for CA19-9-negative such as genetically Lewis-negative subjects as specified elsewhere herein, as a biomarker value below the cutoff is not indicative for absence of the respective condition, e.g. pancreatic cancer, in said subjects. This applies to the personalized cutoff of the biomarker CA19-9 as well. Subjects might be assumed to be biomarker-negative if the biomarker value detemined in samples of said subjects is lower than usually found in subjects not suffering from the respective condition, e.g. lower than 5 U/ml or lower than 2 U/ml for CA19-9.

Therefore, for said subgroup of subjects assumed to be negative for that respective biomarker, e.g. genetically Lewis-negative subjects, a modified algorithm might be used for differentiating between subjects suffering from a condition, e.g. pancreatic cancer, or not. Said modified algorithm is, preferably, determined via a linear regression model, more preferably elastic net, based on the values of the at least two metabolites, e.g. selected from Table 2, as specified elsewere herein, whereby a prediction score is generated by the algorithm. A prediction score cutoff is determined based on a reference data set as specified in detail in the examples. A positive or negative test result is is obtained by comparison of the score, calculated from the at least two metabolites of a sample from a subject, with a score cutoff, determined as specified above. A score of a sample of a subject higer than the respective score cutoff is indicative for the presence of a condition, e.g. pancreatic cancer, whereas a score equal to or lower as the score cutoff is indicative for absence of said condition in said subject. Preferably, the score cutoff is determined by the formula:

$$p = \frac{1}{1 + e^{-(c + \sum_{i=1}^{n} wi \frac{xi - mi}{si})}}$$

where p is the prediction score, c is a specific constant from the model, i is an index of the metabolite, xi are the log-transformed metabolite values, mi and si are feature-specific scaling factors, wi are the coefficients of the model and n is the total number of metabolites.

The determination of the personalized cutoff is, preferably, assisted by automation. For example, a suitable computer program with the algorithm for the calculation of the personalized cutoff and suitable data storage media such as a database, e.g. for storing the determined amounts of the at least two metabolites, the predetermined constants and the calculated personalized cutoff, may be used. Suitable computer programs, algorithms and databases are well known in the art. Notwithstanding the above, the determination of the personalized cutoff can also be carried out manually.

Advantageously, it has been found in the studies underlying the present invention that metabolic analytes can be used for determining a patient-specific individual threshold or personalized cut-off value for a biomarker. By taking into account the individual set-up of the patient, i.e. the amounts of the at least two metabolites as referred to herein, and by providing predetermined constants based on a predefined dataset derived from a diseased patient population and a control population, a personalized cutoff value can be provided. This cutoff value can be used to replace the general standard threshold for a biomarker in a biomarker assay and accordingly, false negative and/or false positive results in a diagnostic assay using said biomarker can be prevented. This is particularly helpful for the diagnosis of heart failure or pancreatic cancer using a personalized cutoff for the biomarkers NT-proBNP or CA 19-9, respectively. The personalized biomarker cutoff described herein, thus, can be directly applied in diagnostic laboratories for each patient. Consequently, the present invention offers an added value for the patient as well as the specialized clinician, leading to a more reliable diagnosis and a better treatment of each individual patient.

The definitions and explanations of the terms made above apply mutatis mutandis for the following embodiments of the present invention except specified otherwise herein below.

In a preferred embodiment of the method, the biomarker is NT-proBNP.

In a further preferred embodiment of the method, the diseased patient population suffers from heart failure and, preferably, from HFrEF.

In another preferred embodiment of the method, the biomarker is CA 19-9.

In still another preferred embodiment of the method, the diseased patient population suffers from pancreatic cancer, more preferably from Pancreatic Ductal Adenocarcinoma (PDAC).

In yet a preferred embodiment of the method, the discrimination comprises linear regression analysis.

In a further preferred embodiment of the method, the determining of a personalized cutoff value for a biomarker in step c) comprises calculating the said cutoff according to formula I:

$$x = c1 + c2 * (c3 - \sum_{i=1}^{n} wi \frac{zi - mi}{si})$$

.

In a yet preferred embodiment of the method, the at least two metabolic analytes are selected from the metabolic analytes of Table 1.

In yet a preferred embodiment of the method, the sample is blood, serum, plasma, urine, salvia, feces or tissue.

In yet a preferred embodiment of the method, the subject is a human.

In yet a preferred embodiment of the method, the amounts of the at least two metabolic analytes are determined by mass spectrometry (MS) or by methods applicable on diagnostic laboratory platforms such as Chemical Analyzers or Immunoanalyzers or combinations of those platforms.

Further encompassed by the present invention is a device for carrying out the method according to the present invention comprising:

a) an analyzing unit comprising at least one detector for at least two metabolic analytes as set forth above, wherein said analyzing unit is adapted for determining the amounts of the said at least two metabolic analytes detected by the at least one detector, and, operatively linked thereto;
b) an evaluation unit comprising a data processor having tangibly embedded a computer program code for carrying out step (b) and step (c) of the method of the present invention.

A device as used herein shall comprise at least the aforementioned units. The units of the device are operatively linked to each other. It will be understood by those skilled in the art that the linkage of the means in an operating manner will depend on the type of units included into the device. For example, where the detector allows for automatic qualitative or quantitative determination of the amounts of the at least two metabolic analytes, the data obtained by said automatically operating analyzing unit can be processed by, e.g., a computer program in order to facilitate the assessment in the evaluation unit. The data obtained by a unit, e.g., the precise amounts of the at least two metabolic analytes, may also be stored in a means capable of storing data. Means and methods to record, store and process data are well known in the art and include, for example, a hard drive, flash drive, CD-R or DVD-R.

Preferably, the units of the device are comprised in a single device. Accordingly, said device may include an analyzing unit for the determination of the amounts of the at least two metabolic analytes and a computer or data processing device as evaluation unit for processing the resulting data for the assessment and for output of the information.

In particular, the evaluation unit shall comprise a data processor having tangibly embedded a computer program code for carrying out step (b) and step (c) of the method according to the present invention. Preferred devices are those which can be applied without the particular knowledge of a specialized clinician, e.g., electronic devices which merely require loading with a sample, preferably a blood, serum or plasma sample. The output information of the device, preferably, is a numerical value, in particular, the personalized cutoff for the biomarker as explained elsewhere herein.

In a preferred embodiment of the present invention, the output information is the personalized cutoff for the biomarker NT-proBNP. As mentioned elsewhere herein, this personalized cutoff value can be used to replace a standard threshold in currently used biomarker assays for the diagnosis of heart failure, e.g. the threshold for the individual patient may be higher or lower than the typical cutoff value of 125 pg/ml, thereby allowing for a more reliable diagnosis whether the individual patient or subject suffers from heart failure or not.

In yet a preferred embodiment of the present invention, the output information is the personalized cutoff for the biomarker CA19-9. As mentioned elsewhere herein, this personalized cutoff value can be used to replace a standard threshold in currently used biomarker assays for the diagnosis of pancreatic cancer, e.g. the threshold for the individual patient may be higher or lower than the typical cutoff value of 37 Units/ml, thereby allowing for a more reliable diagnosis whether the individual patient or subject suffers from pancreatic cancer or not.

Alternatively, the aforementioned units can be implemented into a system comprising several devices which are operatively linked to each other. Operative linkage may be achieved by connecting each device or mean with the other means, i.e. depending on the units to be used for the system of the present invention, which allow data transport in between said means, for example, glass fiber cables, and other cables for high throughput data transport. Moreover, wireless data transfer between the means is also envisaged by the present invention, e.g., via LAN (Wireless LAN, W-LAN). A preferred system comprises means for determining the amounts of at least two metabolic analytes in a sample of a subject. Means for determining the amounts of at least two metabolic analytes as used herein encompass means for separating said at least two metabolic analytes, such as chromatographic devices, and means determination of said at least two metabolic analytes, such as mass spectrometry devices which are described elsewhere herein in detail. Preferred means for metabolic analyte or compound separation to be used according to the present invention include chromatographic devices, more preferably devices for liquid chromatography, HPLC, and/or gas chromatography. Preferred devices for metabolic analyte or compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS are used in the system of the present invention as described elsewhere in detail.

Further comprised shall be means for determining a personalized cutoff for the biomarker based on the results obtained from the means for determining the amounts of said at least two metabolic analytes. Preferably, said determining of the personalized cutoff encompasses a calculation, based on said amounts of the at least two metabolic analytes and said predetermined constants, using an algorithm as explained elsewhere herein in more detail. The means for analyzing the results, i.e. for determining a personalized cutoff value for a biomarker based on the amounts determined in step a) of the method of the present invention and said predetermined constants provided in step (b) of the method of the present invention, shall comprise a data processor having tangibly embedded a computer program code for carrying out said analysis. Moreover, said system may comprise a database and/or further means to record, store and process data. Furthermore, the present invention encompasses the use of said at least two metabolic analytes in a sample of a subject for determining a personalized cutoff value for a biomarker for said subject applying predetermined constants for each of the metabolic analytes which allows for transforming the amounts of the metabolic analytes into a cutoff value for the biomarker, wherein said predetermined constants being derived from discrimination between a diseased patient population and a control population.

Moreover, encompassed by the present invention is a method for diagnosing a disease which is indicated by a biomarker in a subject, comprising the steps of:

(a) determining the amount of at least two metabolic analytes in a sample of a subject;
(b) providing of predetermined constants for each of the metabolic analytes which allows for transforming the determined amounts of the metabolic analytes into a cutoff value for the biomarker, wherein said predetermined constants being derived from discrimination between a diseased patient population and a control population;
(c) determining a personalized cutoff value for a biomarker based on said amounts determined in step a) and said predetermined constants provided in step (b);
(d) providing the amount of the biomarker in a sample of a subject;
(e) comparing said amount provided in step (d) to said personalized cutoff value determined in step (c); and
(f) diagnosing the disease based on said comparison.

In an additional embodiment of the above mentioned method for diagnosing a disease, the amount of the biomarker in a sample of a subject is a previously determined amount.

In a yet preferred embodiment of the above mentioned method for diagnosing a disease, step d) comprises determining the amount of the biomarker in a sample of a subject.

In a yet preferred embodiment of the above mentioned method for diagnosing a disease, the disease is heart failure and, preferably, HFrEF.

In a yet preferred embodiment of the above mentioned method for diagnosing a disease, the disease is pancreatic cancer, more preferably the disease is PDAC.

The term "diagnosing" as used herein refers to assessing whether a subject suffers from a disease or not. Accordingly, the presence or the absence of a disease in the subject can be diagnosed. Preferably, the term refers to ruling in or ruling out said disease. As will be understood by those skilled in the art, such an assessment, although preferred to be, may usually not be correct for 100% of the investigated subjects. The term, however, requires that a statistically significant portion of subjects can be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, and so on. Details can, for example, be found in Dowdy and Wearden, Statistics for Research, John Wiley & Sons, New York 1983. Preferred confidence confidence intervals are at least 50%, at least 60%, at least 70%, at least 80%, at least 90% or at least 95%. The p-values are, preferably, 0.2 or lower, 0.1 or lower, or 0.05 or lower.

Preferably, the diagnosis is based on the personalized cutoff value for said biomarker to be determined in the method of the present invention as explained elsewhere herein in more detail.

The term "diagnosing" includes individual diagnosis of a disease or its symptoms as well as continuous monitoring of a patient. Monitoring, i.e. diagnosing the presence or absence of a disease or the symptoms accompanying it at various time points, includes monitoring of patients known to suffer from the disease as well as monitoring of subjects known to be at risk of developing the disease, e.g. subjects with a genetic predisposition for the disease. Furthermore, monitoring can also be used to determine whether a patient is treated successfully or whether at least symptoms of the disease can be ameliorated over time by a certain therapy. Moreover, monitoring may be used for active patient management including deciding on hospitalization, intensive care measures and/or additional qualitative monitoring as well as quantitative monitoring measures.

The term "disease" as used herein refers to a disease or disorder which, preferably, is indicated by a biomarker in a subject suspected to suffer therefrom. What is meant by the term "biomarker" is described elsewhere in the specification in more detail. In a preferred embodiment of the present invention, said disease is heart failure. In a further preferred embodiment of the present invention, said disease is pancreatic cancer.

As described above, heart failure as used herein relates to an impaired function of the heart. It is a progressive disorder in which the heart fails to pump oxygenated blood at a rate sufficient to meet the metabolic needs of the tissues. Preferably, the term heart failure as used herein relates to congestive heart failure (CHF). Heart failure is known in the art as the final common stage of many cardiovascular diseases and is typically defined as a clinical syndrome in which patients in the final stage show typical signs and symptoms of effort intolerance and/or fluid retention resulting from an abnormality of cardiac structure or function. As outlined further herein below, heart failure in the context of the present invention encompasses both symptomatic forms and asymptomatic forms of heart failure. Accordingly, asymptomatic left ventricular systolic dysfunction or asymptomatic diastolic dysfunction may be diagnosed. Typical symptoms of heart failure include dyspnea, chest pain, dizziness, confusion, pulmonary and/or peripheral edema. Two subsets of heart failure with different pathophysiology are typically described based on a measurement of left ventricular ejection fraction (LVEF), which is the percentage of the total amount of blood in the left ventricle that is pushed out with each heartbeat: Heart failure with reduced left ventricular ejection fraction (HFrEF) and heart failure with preserved left ventricular ejection fraction (HFpEF). Preferably, heart failure as used herein relates to systolic heart failure or heart failure with reduced ejection fraction (HFrEF). It will be understood that the occurrence of the symptoms as well as their severity may depend on the severity of heart failure and the characteristics and causes of the heart failure, systolic or diastolic or restrictive i.e. right or left heart located heart failure. Further symptoms of heart failure are well known in the art and are described in the standard text books of medicine, such as Stedman or Brunnwald.

It will be understood by those skilled in the art that heart failure may be diagnosed by diagnostic assays using the biomarker NT-proBNP as described elsewhere in the specification in more detail. According to the ESC guidance (Ponikowski, 2016), the upper limit of "normal" in the non-acute setting for NT-proBNP is 125 pg/mL (rule-out). In patients with suspected heart failure (symptomatic heart failure) or acute (age dependent) heart failure, the rule out or rule-in settings may be higher or lower, e.g. about 300 pg/ml or >450 pg/ml, >900 pg/ml, >1800 pg/ml for ages <50, 50-75, >75 years, respectively. However, there are currently no personalized biomarker thresholds for each patient and the individual set-up of a patient is currently not considered in diagnostic laboratories. Thus, the biomarker assays with a defined cutoff setting currently used are prone to high rates of false positive or false negative results, i.e. they may wrongly classify a healthy subject as having heaving failure (false positive) or a subject suffering from heart failure as healthy (false negative).

As described above, pancreatic cancer or pancreas cancer as used herein relates to a cancer which is derived from pancreatic cells. Preferably, pancreatic cancer as used herein is pancreatic adenocarcinoma or pancreatic ductal adenocarcinoma (PDAC). Preferably, the pancreatic cancer is a resectable pancreatic cancer, i.e., preferably, is a pancreatic cancer at a tumor stage permitting, preferably complete, resection of the tumor from the subject. More preferably, said pancreatic cancer is a pancreatic cancer of tumor stage IA-IIB. The symptoms accompanying pancreatic cancer are well known from standard text books of medicine such as Stedmen or Pschyrembl and include severe abdominal pain, lower back pain, and in some cases jaundice.

Pancreatic cancer may be diagnosed by diagnostic assays using the biomarker CA19-9 as described elsewhere in the specification in more detail. It is known to those skilled in the art that, however, the level of CA 19-9 may be different between individuals, ranging from non-detectable levels (CA19-9 negative individuals) to patients having relatively high levels of CA 19-9, e.g. due to pre-existing conditions or genetic predispositions. Thus, the guidelines from the American Society of Clinical Oncology discourage the use of CA 19-9 as a general screening test for pancreatic cancer since the rates of false negative or false positive results of the currently available diagnostic assays are rather high. Typically, a cutoff value of 37 Units/ml of CA 19-9 is used for a rule-in or rule-out diagnosis. The individual set-up of a patient is currently not considered in diagnostic laboratories and thus, there are currently no personalized biomarker thresholds for CA19-9 available which would allow for a more reliable diagnosis. As described herein, this problem is solved by the present invention.

The term "determining" or determining the amount" is specified in more detail elsewhere in this specification. In short, determining the amount of the at least two metabolic analytes in a sample of a subject refers to determining at least one characteristic feature of said metabolites in a sample of a subject. Characteristic features in accordance with the present invention, as also described above, are features which characterize the physical and/or chemical properties including biochemical properties of said metabolites. Such properties include, e.g., molecular weight, viscosity, density, electrical charge, spin, optical activity, colour, fluorescence, chemiluminescence, elementary composition, chemical structure, capability to react with other compounds, capability to elicit a response in a biological read-out system (e.g. enzymatic conversion of the metabolites and activation of a dye for colorimetric determination or induction of a reporter gene) and the like. Values for said properties may serve as characteristic features and can be determined by techniques well known in the art. Moreover, the characteristic feature may be any feature which is derived from the values of the physical and/or chemical properties of a metabolite by standard operations, e.g., mathematical calculations such as multiplication, division or logarithmic calculus. Most preferably, the at least one characteristic feature allows the determination and/or chemical identification of said metabolites and their amounts. Accordingly, the characteristic value, preferably, also comprises information relating to the abundance of the metabolite from which the characteristic value is derived. For example, a characteristic value of a metabolite may be an intensity value being related to the abundance of said metabolite (i.e. its amount) in the sample. Means and methods to determine the at least one characteristic feature of a metabolite in a sample of a subject are well known in the art and include, for example, chemical or biological assays such as ELISA, RIA and ECLIA or enzymatic assays or mass spectrometry as referred to elsewhere in the specification in more detail.

The amount of said at least two metabolic analytes in a sample of a subject is, preferably, determined quantitatively or semi-quantitatively in accordance with the present invention. For quantitative determination, either the absolute or precise amount of the metabolites will be determined or the relative amount of the metabolites will be determined based on the value determined for each of the characteristic feature(s) referred to elsewhere herein. Thus, determining the amount of the at least two metabolic analytes encompasses the determination of the absolute amount of the metabolites, the relative amount or concentration of the metabolites in a sample of a subject.

In a preferred embodiment of the method for diagnosing a disease according to the present invention, wherein the disease is heart failure, the amount of the biomarker NT-proBNP is determined in a sample of a subject. In another preferred embodiment of the method for diagnosing a disease according to the present invention, wherein the disease is pancreatic cancer, the amount of the biomarker CA 19-9 is determined in a sample of a subject. It is also envisaged that the amount of said biomarker, preferably of NT-proBNP or CA 19-9, can be derived from the medical record of the subject to be tested. The method of the present invention may, thus, comprise the step of providing and/or retrieving information on the amount of NT-proBNP or CA 19-9 (i.e. the value of this marker). Accordingly, said amount of the biomarker in a sample of a subject might also be a previously determined amount (e.g. a biomarker value derived from the medical record of the subject to be tested).

In a further embodiment of the method for diagnosing a disease according to the present invention, the sample is, preferably, split into at least two subsamples, of which in one subsample the metabolic analytes, preferably small molecule metbolites, are determined and of which in a second subsample the respective biomarker, e.g. NT-proBNP or CA19-9, is determined. Preferably, small molecule metabolites are determined in a first sample and NT-proBNP or CA19-9 is determined in a second sample, wherein, preferably, said samples are taken at the same time, or, in another embodiment of the method, at different times. In a further embodiment of the method for diagnosing a disease according to the present invention, wherein the disease is diagnosing pancreatic cancer, CA19-9 is not determined; in such case, preferably, the subject is a subject known or suspected to be a subject with a low CA19-9 value as specified elsewhere herein, more preferably a subject which is Lewis a/b antigen negative, as specified elsewhere herein. Thus, preferably, in a subject with a known very low CA19-9 value, preferably less than 2 U/mL, and therefore most probably being a Lewis a/b antigen negative subject, the at least two metabolites from Table 2 are applied without CA19-9.

In a preferred embodiment of the method for diagnosing a disease which is indicated by a biomarker in a subject, said personalized cutoff value based on said amounts determined in step a) and said predetermined constants provided in step (b) according to the method of the present invention is a personalized cutoff value for the biomarker NT-proBNP or the biomarker CA 19-9.

The term "comparing" as used herein, preferably, refers to determining whether the determined value, i.e. the personalized cutoff value determined in step (e) of the method for diagnosing a disease of the present invention, is below the measured amount of the biomarker

Based on the comparison referred to above, a subject can be assessed to suffer from a disease. In a preferred embodiment of the present invention said disease is heart failure or pancreatic cancer.

The comparison is, preferably, assisted by automation. For example, a suitable computer program comprising algorithms for the comparison of two different data sets, e.g., data sets comprising values of the characteristic feature(s) including the determined amount of the biomarker and the individual threshold, may be used. Such computer programs and algorithms are well known in the art. Notwithstanding the above, a comparison can also be carried out manually.

All references cited in this specification are herewith incorporated by reference with respect to their entire disclosure content and the disclosure content specifically mentioned in this specification.

**Figure Legends**

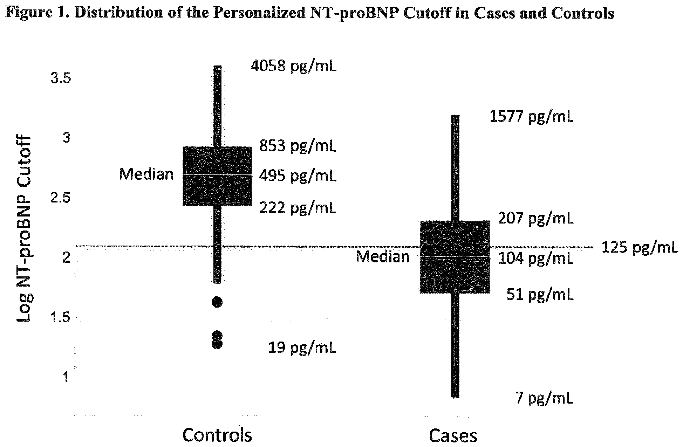

**Figure 1: Distribution of the Personalized NT-proBNP Cutoff in Cases and Controls**
Figure 1 shows the distribution of the personalized cutoff for the biomarker NT-proBNP in human plasma samples of 367 subjects suffering from heart failure with reduced ejection fraction (cases) and of 238 subjects without heart failure (controls), Data are presented as Box plots with log-transformed ordinate. Maximum, 3rd Quantile, Median, 1st Quantile, Minimum values of the personalized cutoff in pg/ml are given for both groups.

**Figure 2: Ranges of NT-proBNP values and corresponding personalized NT-proBNP Cutoffs**

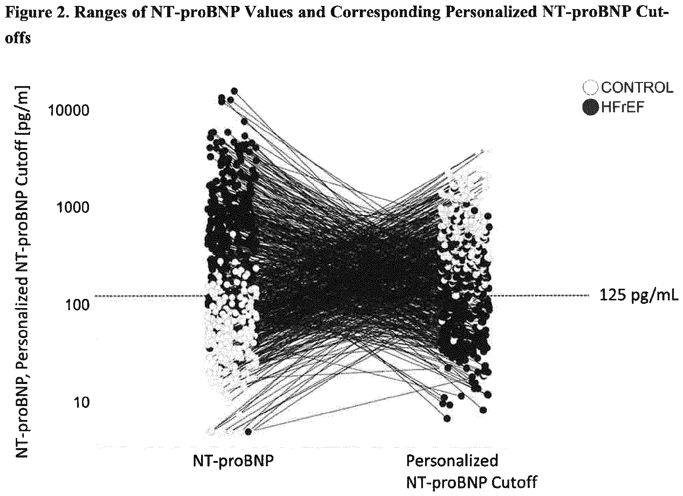

Figure 2 shows the distribution and ranges of the NT-proBNP values and of the personalized cutoff for the biomarker NT-proBNP in human plasma samples of 367 subjects suffering from heart failure with reduced ejection fraction (HFrEF, black dots) and of 238 subjects without heart failure (controls, grey dots). Values of the same sample are connected by a grey line. The general NT-proBNP cutoff is indicated with a dashed line.

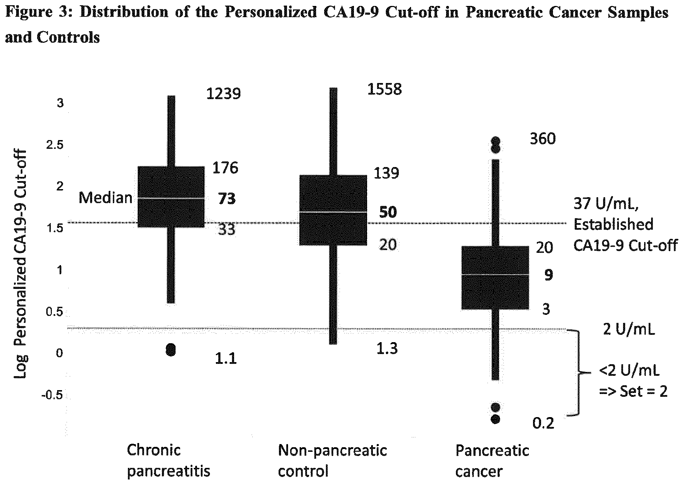

**Figure 3: Distribution of the Personalized CA19-9 Cut-off in Pancreatic Cancer Samples and Controls**
Figure 3 shows the distribution of the personalized cutoff for the biomarker CA19-9 in human plasma samples of 190 subjects suffering from pancreatic cancer, 132 subjects suffering from chronic pancreatitis and 182 controls without pancreatic disease. Data are presented as Box plots with log-transformed ordinate. Maximum, 3rd Quantile, Median, 1st Quantile, Minimum values of the personalized cutoff in pg/ml are given for all three groups. The general cutoff for CA19-9 of 37 U/mL is indicated by a grey line. The threshold for the personalized CA19-9 cutoff of 2 U/mL discriminating between CA19-9 positive and negative subjects is indicated by a dashed line. U, units.

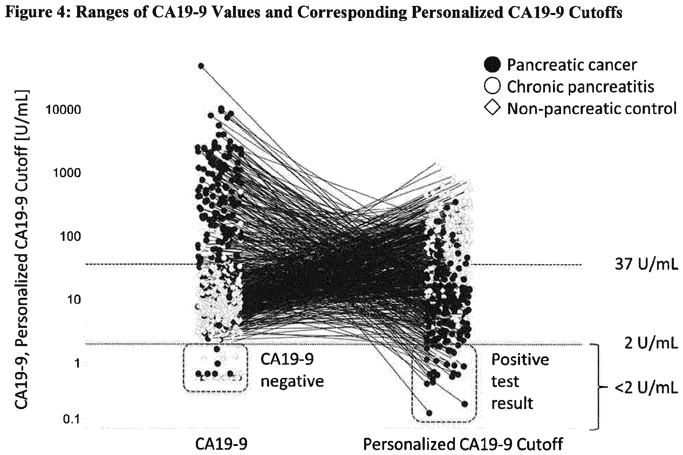

**Figure 4: Ranges of CA19-9 Values and Corresponding Personalized CA19-9 Cutoffs**
Figure 4 shows the distribution and ranges of the CA19-9 values and of the personalized cutoff for the biomarker CA19-9 in human plasma samples of 190 subjects suffering from pancreatic cancer, 132 subjects suffering from chronic pancreatitis and 182 controls without pancreatic disease. Values of the same sample are connected by a grey line. The general CA19-9 cutoff of 37 U/mL is indicated with a dashed line. The threshold of 2 U/mL discriminating between CA19-9 positive and negative subjects is indicated by a grey line. U, units.

**EXAMPLES**

The invention will now be illustrated by the following Examples which are not intended to restrict or limit the scope of this invention.

**Example 1: Analytical protocol for the analysis of metabolic analytes**

Sample extraction and HPLC-MS/MS system:

10 μl plasma were mixed with 1500 μl extraction solvent containing methanol/dichloromethane (in a ratio of 2:1, v/v) and 10 μl internal standard mixture in a 2 ml safelock microcentrifuge tube (Eppendorf, Germany). The internal standard solution contained 41.28 μg/ml phosphatidylcholine (PC) 19:0/19:0 (Avanti Polar Lipids, CA, U.S.A.) in extraction solvent. Ultrapure water (Milli-Q water system, Millipore) and analytical grade chemicals were used for extraction, dilution or as LC solvents. Quality control and reference sample were prepared from commercially available human plasma (RECIPE Chemicals + Instruments GmbH). Delipidized plasma (Plasma, Human, Defibrinated, Delipidized, 2X Charcoal treated, Highly Purified; USBio) was used for the preparation of calibrators and blanks. After thoroughly mixing at 20°C for 5min, the precipitated proteins were removed by centrifugation for 10 min. An aliquot of the liquid supernatant was transferred to an appropriate glass vial and stored at -20°C until analysis by LC-MS/MS. Up to 5 μL of the crude extract were injected into an HPLC-MS/MS systems consisting of an Agilent 1100 LC system (Agilent Technologies, Waldbronn, Germany) coupled to an AB-Sciex™ API 4000 triple quadrupole mass spectrometer (ABSCIEX, Toronto, Canada). The method is intended to be compatible with e.g. the ABSciex™ 3200MD benchtop LC-MS/MS system.

Liquid chromatography gradient:

HPLC analysis was performed at 55°C on commercially available reversed phase separation columns with C18 stationary phases (Ascentis® Express C18 column (5 cm × 2.1 mm, 2.7 μm, Phenomenex, Germany) using a gradient of Solvent A (methanol, water, formic acid, 400:400:1, w/w/w) against Solvent B (tert-butyl methyl ether, 2-propanol, methanol, formic acid, 400:200:100:1, w/w/w). Solvent C (methanol, 0.1 M ammonium formate solution in water, 20:3, w/w) was added post-column during the elution time of triglycerides. Details of the gradient are given in Suppl. Table 1A below.

Table 3A: Liquid chromatography gradient used in the dedicated analytical protocol

| Analytical Gradient | | | | Post-Column Addition | | |
|---|---|---|---|---|---|---|
| Time [min] | Flow Rate [μl/min] | Solvent A [%] | Solvent B [%] | Time [min] | Flow [μl/min] | Solvent C [%] |
| 0.00 | 400 | 100 | 0 | 0.00 | 0 | 100 |
| 0.10 | 400 | 100 | 0 | ... gradient continued... | | |
| 0.20 | 400 | 100 | 0 | | | |
| 0.40 | 500 | 58 | 42 | | | |
| ... gradient continued... | | | | 3.20 | 0 | 100 |
| 3.30 | 500 | 32 | 68 | 3.30 | 200 | 100 |
| 5.00 | 600 | 15 | 85 | ... gradient continued... | | |
| ...gradient continued... | | | | 5.30 | 200 | 100 |
| | | | | 5.40 | 0 | 100 |
| 5.50 | 600 | 15 | 85 | ...gradient continued... | | |
| 5.70 | 400 | 100 | 0 | | | |
| 7.50 | 400 | 100 | 0 | 7.50 | 0 | 100 |

Mass spectrometry using multiple-reaction-monitoring (MRM):

The source parameters were: nebulizer gas, 50; heater gas, 60; curtain gas, 25; CAD gas, 4; ion spray voltage, 5500 V; temperature, 400°C; pause between mass ranges, 5 ms; resolution Q1 and Q3, unit. MRM parameters, retention times, and molecular species are listed in Suppl. Table 3B below.

Table 3B: Retention times and MRM settings of the LC-MS/MS analysis used in the dedicated analytical protocol

| Metabolomic feature | Dwell Time [msec] | Q1 [Da] | Q3 [Da] | DP [V] | EP [V] | CE [V] | CXP [V] | RT [min] |
|---|---|---|---|---|---|---|---|---|
| PC 16:0/18:2 | 10 | 758.6 | 184.1 | 85/60* | 10 | 70/85* | 15 | 1.72 |
| Sum of SM d18:1/23:1 SM d18:2/23:0 SM d17:1/24:1 | 10 | 799.5 | 184.1 | 85/60* | 10 | 40 | 15 | 2.02 |
| PC 19:0/19:0 (internal standard for SM, TAG, and CE, collision energy 40V) | 10 | 818.6 | 184.1 | 85/60* | 10 | 40 | 15 | 2.38 |
| PC 19:0/19:0 (internal standard for PC, collision energy 70V) | 10 | 818.6 | 184.1 | 85/60* | 10 | 70/85* | 15 | 2.38 |
| TAG 18:1/18:0/18:0 | 10 | 906.9 | 605.4 | 75/30* | 10 | 35 | 10 | 4.87 |

MRM settings (Q1, first mass transition; Q3, second mass transition; DP, declustering potential; EP, entrance potential; CE, collision energy; CXP, collision cell exit potential) and retention time (RT) for each metabolite. * <setting used for non-quantitative determination>/<setting used for quantitative determination>

Absolute quantification:

Absolute quantification was achieved using commercially available phosphatidylcholine, sphingomyelin, and triglyceride species as external standards. The MRM settings were changed as shown in Table 3B (indicated by *) to optimize the linear range of the measurements.

**Example 2: Calculation of the personalized NT-proBNP cutoff**

In a case-control study with samples of subjects suffering from heart failure with reduced ejection fraction (HFrEF) and of controls without heart failure the personalized cutoff was calculated as:

$$z = c1 + c2 * \left(c3 - \sum_{i=1}^{n} wi \frac{xi - mi}{si}\right)$$

where z is the log10 transformed personalized cutoff, while c1, c2, and c3 are specific constants, $x_i$ are the log-transformed metabolite analytical results taking into account the units listed in Table 4A, $m_i$ and $s_i$ are feature-specific scaling factors, and w, are the coefficients of the model, n, total number of features in the panel.

Table 4A: Features, scaling factors and coefficients used for the calculation of the personalized NT-proBNP cutoff for discrimination between HFrEF patients and controls (input data for the metabolic features in the form of LC-MS/MS peak area ratios relative to the internal standard PC 19:0/19:0). The scaling factors mi and si were adjusted rather than the coefficients wi because it allows the logistic regression model with the coefficients wi to remain independent of the analytical method used.

| Feature | Index $i$ | Scaling Factors | | Coefficients | Constants |
|---|---|---|---|---|---|
| | | $m_i$ | $s_i$ | $w_i$ | $c$ |
| c1 | | | | | 2.20238 |
| c2 | | | | | 0.4042091 |
| c3 | | | | | 0.2753094 |

(continued)

| Feature | Index $i$ | Scaling Factors | | Coefficients | Constants |
|---|---|---|---|---|---|
| | | $m_i$ | $s_i$ | $w_i$ | $c$ |
| Sum of SM d18:1/23:1, d18:2/23:0, and d17:1/24:1 | 1 | -0.5379916 | 0.115942 | -0.7158906 | |
| TAG 18:1/18:0/18:0 | 2 | -2.1316954 | 0.3863649 | 0.6762354 | |
| PC 16:0/18:2 | 3 | 0.8736775 | 0.0834807 | -0.4594183 | |

In an additional study with subjects suffering from pulmonary disease the scaling factors xi and mi were adjusted for the use of absolute concentration as input data (Table 4B). The coefficients wi were not changed.

Table 4B: Features, scaling factors and coefficients used for the calculation of the personalized cutoff (input data for the metabolic features xi in the form of absolute concentrations in µg/dl based on external calibration standards).

| Feature | Index $i$ | Scaling Factors | | Coefficients | Constants |
|---|---|---|---|---|---|
| | | $m_i$ | $s_i$ | $w_i$ | $c$ |
| c1 | | | | | 2.20238 |
| c2 | | | | | 0.4042091 |
| c3 | | | | | 0.2753094 |
| Sum of SM d18:1/23:1, d18:2/23:0, and d17:1/24:1 | 1 | 3.106761 | 0.123062 | -0.7158906 | |
| TAG 18:1/18:0/18:0 | 2 | 2.12519 | 0.3926955 | 0.6762354 | |
| PC 16:0/18:2 | 3 | 4.583751 | 0.0892658 | -0.4594183 | |

$$z = c1 + c2 * (c3 - \sum_{i=1}^{n} wi \frac{xi - mi}{si})$$

Example calculation:

Personalized biomarker cutoff (NT-pro BNP):

$$z=2.20238+0.4042091*(0.2753094+(0.7158906*((x2-3.106761)/0.123062))-(0.6762354*((x3-2.12519)/0.3926955))+(0.4594183*((x4-4.583751)/0.0892658)))$$

Table 4C shows increased AUC, specificity and PPV of the personalized NT-proBNP cutoff compared to the general cutoff of 125 pg/ml in a study with 371 subjects with heart failure with reduced ejection fraction and 244 subjects without heart failure; AUC, area under the curve, PPV, positive predictive value, NPV, negative predictive value.

Table 4C: Improved Specificity of the Personalized NT-proBNP Cutoff

| NT-proBNP | AUC | Sensitivity | Specificity | PPV* | NPV* |
|---|---|---|---|---|---|
| General Cutoff, 125 pg/ml | 0.928 | 0.803 | **0.869** | **0.244** | 0.988 |
| Personalized Cutoff | 0.970 | 0.795 | **0.984** | **0.719** | 0.989 |
| *Based on 5% Prevalence | | | | | |

Table 4D illustrates how the personalized NT-proBNP cutoff defines the reference range for the NT-proBNP value of a subject's sample.

Table 4D: Examples Result Output for NT-proBNP with a Personalized Reference Range

| (1) | | | |
|---|---|---|---|
| **Analyte** | **Result** | **Unit** | **Reference Range** |
| (a) Personalized NT-proBNP Cutoff | 561 | pg/mL | |
| **(b) NT-proBNP** | **52** | **pg/mL** | **NEW: < 561** |

| | | | |
|---|---|---|---|
| (2) | | | |
| **Analyte** | **Result** | **Unit** | **Reference Range** |
| (a) Personalized NT-proBNP Cut-off | 1886 | pg/mL | |
| **(b) NT-proBNP** | **284** | **pg/mL** | **NEW: < 1886** |
| (3) | | | |
| **Analyte** | **Result** | **Unit** | **Reference Range** |
| (a) Personalized NT-proBNP Cut-off | 71 | pg/mL | |
| **(b) NT-proBNP** | **622** | **pg/mL** | **NEW: < 71** |

| | | | |
|---|---|---|---|
| (4) | | | |
| **Analyte** | **Result** | **Unit** | **Reference Range** |
| (a) Personalized NT-proBNP Cut-off | 34 | pg/mL | |
| **(b) NT-proBNP** | **61** | **pg/mL** | **NEW: < 34** |

Table 4E summarizes the subject classes and test outcomes, Non-HF, subject without heart failure, HFrEF, subject with heart failure with reduced ejection fraction.

Table 4E: Summary of test outcomes

| Subject | Test result |
|---|---|
| Non-HF | True negative, sample NT-proBNP concentration is lower than the personalized cut-off |
| Non-HF | True negative, sample NT-proBNP concentration is lower than the personalized NT-proBNP cutoff of that subject, though higher than the general cutoff of 125 pg/mL |
| HFrEF | True positive, sample NT-proBNP concentration is higher than the personalized cut-off |
| HFrEF | True positive, sample NT-proBNP concentration is higher than the personalized NT-proBNP cutoff of that subject, though lower than the general cutoff of 125 pg/mL |

**Example 3: Analytical method for analysis of metabolites, e.g. from Table 2, for use of determining a personalized CA19-9 cutoff**

Human plasma samples were prepared and subjected to LC-MS/MS analysis as follows: 20 µl human plasma was mixed with 100 µl internal standard mixture (alanine d4: 12.24 µg/ml; ceramide (d18:1,C17:0): 0.154 µg/ml were dissolved in dimethyl sulfoxide, methanol, dichloromethane and water (in a ratio 12.3 : 2.2 : 1.1 : 1, v/v/v/v)) and 700 µl extraction solvent containing methanol and dichloromethane in a ratio of 2 : 1 (v/v).
After the samples were thoroughly mixed at 20°C for 5 min, the precipitated proteins were removed by centrifugation for 10 min. 150 µl of the liquid supernatant was transferred to an appropriate glass vial for further derivatization with dansyl chloride, which allows the dansylation of primary and secondary amine groups. For this purpose, 25 µl of 0.2 mol/l sodium bicarbonate buffer (dissolved in water), 25 µl of 4 mg/ml dansyl chloride solution (dissolved in acetonitrile) and 50 µl dimethyl sulfoxide were added. The dansylation was carried out under constant mixing at 35°C for 150 min.

The so- obtained reaction mixtures were analyzed by LC-MS/MS.

The LC-MS/MS systems consisted of an Agilent 1100 LC system (Agilent Technologies, Waldbronn, Germany) coupled with an API 4000 Mass spectrometer (ABSCIEX, Toronto, Canada). HPLC analysis was performed on commercially available reversed phase separation columns with C18 stationary phases (Phenomenex Ascentis Express C18, 2.7 $\mu$m, 50 x 2.1 mm).

Up to 2 $\mu$l of the above-mentioned so-obtained reaction mixture was injected and separated by gradient elution using a mixture of solvents consisting of methanol, water, formic acid, 2-propanol and 2-methoxy-2-methylpropane at a flow rate of 600 $\mu$l/min (e.g. starting from 0% solvent B to 100% solvent B in 7 min):

Solvent A: 400 g methanol, 400 g water, 1 g formic acid
Solvent B: 400 g 2-methoxy-2-methylpropane, 200 g 2-propanol, 100 g methanol, 1 g formic acid

Mass spectrometry was carried out by electrospray ionization (ESI) in positive ion mode using multiple reaction monitoring (MRM). Using ESI, sphingomyelins with equal numbers of carbons and double bonds were detected together, these isobaric species were not separated chromatographically.

The diagnostic biomarkers listed in Table 2 can be measured with MRM. The respective amino acid analytes were measured with a quantifier and a qualifier MRM transition, whereas the analytes of the diagnostic biomarkers listed in Table 1 are measured with a respective quantifier only.

Quantitative evaluations of all small molecule biomarkers with commercially available quantification standards were achieved by external calibration in delipidized plasma. Delipidized plasma was used to simulate a matrix as close as possible to real plasma. For small molecule biomarkers without commercially available standards, a commercially available standard of the same lipid class was used for the external calibration.

Reference controls were prepared by lyophilization of different amounts of commercially available human plasma (12 $\mu$l, 20 $\mu$l, 28 $\mu$l) to check the linearity of small molecule biomarkers under real matrix condition. The ratios of the calculated concentrations of 12 $\mu$l / 20 $\mu$l and 28 $\mu$l / 20 $\mu$l of the lyophilized human reference control plasma delivered values between 0.5 and 0.7 and between 1.3 and 1.5, respectively, for all small molecule biomarkers of Table 1.

Recovery controls were prepared by adding a known standard concentration to lyophilized plasma samples of the same commercially available human reference control plasma as used for the reference controls. The lyophilized plasma samples were stored in a freezer until sample preparation.

Inter-day quality controls were prepared by extracting multiple samples of the commercially available human reference control plasma with extraction solvent and internal standard solution followed by dansylation. The dansylated reaction mixtures of all samples were pooled, and stored in aliquots in a freezer until they were used for the daily quality control of the instrument performance and sample preparation.

Quality controls for the method precision were prepared daily by extracting multiple samples of the commercially available human reference control plasma and were measured equally distributed in the sample batch.

**Example 4: Calculation of a personalized CA19-9 cutoff**

In this example the features of the algorithm for calculation of the personalized CA19-9 cutoff were derived from the metabolite and CA19-9 values of samples from subjects suffering from pancreatic cancer or suffering from chronic pancreatitis or from non-pancreatic controls. A classifier of pancreatic cancer diagnosis was obtained by training the elastic net algorithm on the predefined panels as described by Zou and Hastie ((2005) Regularization and variable selection via the elastic net, Journal of the Royal Statistical Society, Series B, 67, 301-320) using the pancreatic cancer group as positive class and the chronic pancreatitis and/or the non-pancreatic controls as negative class, respectively. In order to reduce overfitting effects, tenfold cross-validation was done which is well known to a person skilled in the art. As a result, for each of the classifiers weightings and scalings are obtained that are optimized for the classes they were trained for.

Those classifiers are subsequently tested by the same and further classification tasks, those being pancreatic cancer relative to chronic pancreatitis, pancreatic cancer relative to non-pancreatic controls, pancreatic cancer relative to (chronic pancreatitis and nonpancreatic controls), pancreatic cancer relative to all non-cancer subjects (chronic pancreatitis, non-pancreatic controls, diabetes group, non-diabetes group, and pancreatic cancer relative to diabetic subjects (from chronic pancreatitis, non-pancreatic controls, diabetes group).

The personalized cutoff was calculated as:

$$z = c1 + c2 * (c3 - \sum_{i=1}^{n} wi \frac{xi - mi}{si})$$

where z is the log10 transformed personalized cutoff, while c1, c2, and c3 are specific constants, $x_i$ are the log-transformed metabolite analytical results taking into account the units listed in Tables 5A or 5B, $m_i$ and $s_i$ are feature-specific scaling factors, and $w_i$ are the coefficients of the model and $n$ is the total number of features in the metabolite panel.

Table 5A: Features, scaling factors and coefficients used for the calculation of the personalized CA19-9 cutoff for subjects positive for CA19-9 (input data for the metabolic features in the form of absolute concentrations based on external calibration standards).

| Feature | Index $i$ | Scaling Factors $m_i$ | $s_i$ | Coefficients $w_i$ | Constants |
|---|---|---|---|---|---|
| c1 | | | | | 1.703977 |
| c2 | | | | | 0.6537 |
| c3 | | | | | -0.7666746 |
| Proline | 1 | 3.208503 | 0.1514107 | -0.3291846 | |
| Tryptophan | 2 | 2.876533 | 0.1527579 | -0.2227659 | |
| Lysophosphatidylethanolamine (C18:2) | 3 | 1.723299 | 0.2621586 | -0.25298 | |
| Sphingomyelin (d18:2,C17:0) | 4 | 1.766133 | 0.1692918 | 0.1783197 | |
| Sphingomyelin (d17:1,C18:0) | 5 | 2.491948 | 0.1761938 | 0.6867066 | |
| Ceramide (d18:1,C24:0) | 6 | 2.354364 | 0.1721005 | -0.3639318 | |

For generation of the test result, the calculated personalized CA19-9 cutoff is compared with the CA19-9 value of the respective subject, in case the calculated personalized cutoff is higher than CA19-9 values commonly found in samples of Lewis-negative subjects, such as e.g. 2 Units/mL. In case of a calculated personalized CA19-9 cutoff equal as or lower than that threshold of e.g. 2 Units/mL, the test output is positive irrespective of the CA19-9 value of said subject.

It is well known in the art that a biomarker cannot be reasonably used in samples of subjects known or assumed to be negative for that respective biomarker, e.g. CA19-9 for CA19-9-negative such as genetically Lewis-negative subjects as specified elsewhere herein, as a biomarker value below the cutoff is not indicative for absence of the respective condition, e.g. pancreatic cancer, in said subjects. This applies to the personalized cutoff of said biomarker as well. Subjects might be assumed to be biomarker-negative if the biomarker value detemined in samples of said subjects is lower than usually found in subjects not suffering from the respective condition, e.g. lower than 5 U/ml or lower than 2 U/ml for CA19-9.

Therefore, for said subgroup of subjects, a modified algorithm might be used for differentiating between subjects suffering from a condition, e.g. pancreatic cancer, or not. Said modified algorithm is, preferably, determined via a linear regression model, more preferably elastic net, based on the values of the at least two metabolites, e.g. selected from Table 2, as specified elsewere herein, whereby a prediction score is generated by the algorithm. A prediction score cutoff is determined based on a reference data set as specified in detail in the examples. A positive or negative test result is is obtained by comparison of the score, calculated from the at least two metabolites of a sample from a subject, with a score cutoff, determined as specified above. A score of a sample of a subject higer than the respective score cutoff is indicative for the presence of a condition, e.g. pancreatic cancer, whereas a score equal to or lower as the score cutoff is indicative for absence of said condition in said subject. Preferably, the score cutoff is determined by the formula:

$$p = \frac{1}{1 + e^{-(c + \sum_{i=1}^{n} wi \frac{xi - mi}{si})}}$$

where p is the prediction score, c is a specific constant from the model, i is an index of the metabolite, $x_i$ are the log-transformed metabolite values, $m_i$ and $s_i$ are feature-specific scaling factors, $w_i$ are the coefficients of the model and $n$ is the total number of metabolites.

Table 5B: Features, scaling factors and coefficients used for the calculation of a score calculated from the at least two metabolites listed in Table 2 and indicative for pancreatic cancer in subjects known or suspected to be CA19-9-negative, such as e.g. Lewis-negative subjects, e.g. subjects with CA19-9 values equal or lower than e.g. 2 Units/mL.

| Feature | Index $i$ | Scaling Factors $m_i$ | $s_i$ | Coefficients $w_i$ | Constants $c$ |
|---|---|---|---|---|---|
| c | | | | | - 0.0365877 |
| Proline | 1 | 3.207728 | 0.1528619 | - 0.27840448 | |
| Tryptophan | 2 | 2.873306 | 0.1552379 | - 0.07395472 | |
| Lysophosphatidylethanolamine (C18:2) | 3 | 1.720551 | 0.2612813 | - 0.34913978 | |
| Sphingomyelin (d18:2,C17:0) | 4 | 1.763707 | 0.1675896 | 0.23013161 | |
| Sphingomyelin (d18:1,C17:0) | 5 | 2.4896 | 0.1730836 | 0.58721148 | |
| Ceramide (d18:1,C24:0) | 6 | 2.354094 | 0.1698166 | - 0.31900718 | |

The score was calculated by the formula

$$p = \frac{1}{1 + e^{-(c + \sum_{i=1}^{n} wi \frac{xi - mi}{si})}}$$

where p is the prediction score, c is a specific constant from the model, i is an index of the metabolite, $x_i$ are the log-transformed metabolite values, $m_i$ and $s_i$ are feature-specific scaling factors, $w_i$ are the coefficients of the model and $n$ is the total number of metabolites.

Table 5C shows increased AUC, specificity and PPV of the personalized NT-proBNP cutoff compared to the general cutoff of 37 U/ml in a validation study with 103 subjects with resectable pancreatic adenocarcinoma and 94 subjects with chronic pancreatitis; AUC, area under the curve.

Table 5C: Improved Sensitivity of the Personalized CA19-9 Cutoff

| CA19-9 | AUC | Sensitivity | Specificity |
|---|---|---|---|
| General Cutoff, 37 U/mL | 0.88 | **0.68** | 0.87 |
| Personalized Cutoff | 0.93 | **0.83** | 0.87 |

CITED REFERENCES

Collerton et al, 2014. Utility of NT-proBNP as a rule-out test for left ventricular dysfunction in very old people with limiting dyspnoea: the Newcastle 85+ Study. BMC cardiovascular disorders, 14(1), 128.

Fry et al., 2008. Molecular markers of pancreatic cancer: development and clinical relevance. Langenbeck's archives of surgery, 393(6), 883-890.

Ponikowski et al, 2016. ESC Guidelines for the diagnosis and treatment of acute and chronic heart failure: The Task Force for the diagnosis and treatment of acute and chronic heart failure of the European Society of Cardiology (ESC) Developed with the special contribution of the Heart Failure Association (HFA) of the ESC. European heart journal, 37(27), 2129-2200).

WO 03/073464 A1, METANOMICS GMBH & CO KGAA, Mass spectrometry method for analysing mixtures of substances.

Mueller-Hennessen et al., 2016 "A novel lipid biomarker panel for the detection of heart failure with reduced ejection fraction." Clinical chemistry (2016): clinchem-2016.

Wahlefeld AW, Bergmeyer HU, eds. Methods of Enzymatic Analysis. 2nd English ed. New York, NY: Academic Press Inc 1974; 1831

**Claims**

1. A method for determining a personalized cutoff value for a biomarker comprising the steps:

    (a) determining in a sample of a subject the amounts of at least two metabolic analytes;
    (b) providing predetermined constants which allow for transforming the determined amounts of the metabolic analytes into a cutoff value for the biomarker, wherein said predetermined constants being derived from discrimination between a diseased patient population and a control population based on the metabolic analytes; and
    (c) determining a personalized cutoff value for a biomarker based on the amounts determined in step a) and said predetermined constants provided in step (b).

2. The method of claim 1, wherein said biomarker is NT-proBNP or CA 19-9.

3. The method of claims 1 or 2, wherein said diseased patient population suffers from heart failure or pancreatic cancer and, preferably, from HFrEF or PDAC.

4. The method of any one of claims 1 to 3, wherein said discrimination comprises linear regression analysis.

5. The method of any one of claims 1 to 4, wherein said determining a personalized cutoff value for a biomarker in step c) comprises calculating the said cutoff according to formula I:

$$x = c1 + c2 * (c3 - \sum_{i=1}^{n} wi \frac{zi - mi}{si})$$

.

6. The method of any one of claims 1 to 5, wherein said at least two metabolic analytes are selected from the metabolic analytes of Table 1.

7. The method of any one of claims 1 to 6, wherein the sample is blood, serum, plasma, urine, salvia, feces or tissue.

8. The method of any one of claims 1 to 7, wherein the subject is a human.

9. The method of any one of claims 1 to 8, wherein the amounts of the at least two metabolic analytes are determined by mass spectrometry (MS) or by methods applicable on diagnostic laboratory platforms such as Chemical Analyzers or Immunoanalyzers or combinations of those platforms.

10. A device for carrying out the method according to any one of claims 1 to 9 comprising:

    a) an analyzing unit comprising at least one detector for determining the amounts of the at least two metabolic analytes as set forth in any one of claims 1 to 9, and operatively linked thereto;
    b) an evaluation unit comprising a data processor having tangibly embedded a computer program code for carrying out step (b) and step (c) of the method of any one of claims 1 to 9.

11. Use of at least two metabolic analytes as set forth in any one of claims 1 to 9 in a sample of a subject for determining a personalized cutoff value for a biomarker for said subject applying predetermined constants which allow for transforming the amounts of the metabolic analytes into a cutoff value for the biomarker, wherein said predetermined constants being derived from discrimination between a diseased patient population and a control population.

12. A method for diagnosing a disease which is indicated by a biomarker in a subject comprising the steps of:

    (a) determining the amount of at least two metabolic analytes in a sample of a subject;
    (b) providing of predetermined constants which allow for transforming the determined amounts of the metabolic analytes into a cutoff value for the biomarker, wherein said predetermined constants being derived from discrimination between a diseased patient population and a control population;
    (c) determining a personalized cutoff value for a biomarker based on said amounts determined in step a) and said predetermined constants provided in step (b);
    (d) providing the amount of the biomarker in a sample of a subject;

(e) comparing said amount provided in step (d) to said personalized cutoff value determined in step (c); and

(f) diagnosing the disease based on said comparison.

13. The method of claim 12, wherein said amount of the biomarker in a sample of a subject is a previously determined amount.

14. The method of claim 12, wherein step d) comprises determining the amount of the biomarker in a sample of a subject.

15. The method of any one of claims 12 to 14, wherein said disease is heart failure or pancreatic cancer and, preferably, HFrEF or PDAC.

Figure 1. Distribution of the Personalized NT-proBNP Cutoff in Cases and Controls

**Figure 2. Ranges of NT-proBNP Values and Corresponding Personalized NT-proBNP Cut-offs**

**Figure 3: Distribution of the Personalized CA19-9 Cut-off in Pancreatic Cancer Samples and Controls**

**Figure 4: Ranges of CA19-9 Values and Corresponding Personalized CA19-9 Cutoffs**

| | Europäisches Patentamt European Patent Office Office européen des brevets | **EUROPEAN SEARCH REPORT** | **Application Number** EP 19 15 7826 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | STEFAN ENROTH ET AL: "Strong effects of genetic and lifestyle factors on biomarker variation and use of personalized cutoffs", NATURE COMMUNICATIONS, vol. 5, 22 August 2014 (2014-08-22), page 4684, XP055209636, DOI: 10.1038/ncomms5684 * throughout, e.g. abstract, pgs. 4-6 * | 1-15 | INV. G16H50/20 G16B40/00 G16B20/20 |
| X | GIANLUIGI SAVARESE ET AL: "Associations With and Prognostic and Discriminatory Role of N-Terminal Pro-B-Type Natriuretic Peptide in Heart Failure With Preserved Versus Mid-range Versus Reduced Ejection Fraction", JOURNAL OF CARDIAL FAILURE., vol. 24, no. 6, 1 June 2018 (2018-06-01), pages 365-374, XP55609310, US ISSN: 1071-9164, DOI: 10.1016/j.cardfail.2018.03.010 * throughout, e.g. abstract, discussion * | 1-15 | |
| | | | TECHNICAL FIELDS SEARCHED (IPC) G16H G16B |

The present search report has been drawn up for all claims

| Place of search | Date of completion of the search | Examiner |
|---|---|---|
| Munich | 31 July 2019 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4540884 A
- US 5397894 A
- WO 03073464 A
- WO 03073464 A1

**Non-patent literature cited in the description**

- **FRY et al.** Molecular markers of pancreatic cancer: development and clinical relevance. *Langenbeck's archives of surgery,* 2008, vol. 393 (6), 883-890
- **PONIKOWSKI et al.** ESC Guidelines for the diagnosis and treatment of acute and chronic heart failure: The Task Force for the diagnosis and treatment of acute and chronic heart failure of the European Society of Cardiology (ESC) Developed with the special contribution of the Heart Failure Association (HFA) of the ESC. *European heart journal,* 2016, vol. 37 (27), 2129-2200
- **WAHLEFELD et al.** Methods of Enzymatic Analysis. Academic Press Inc, 1974, 1831
- **NIESSEN et al.** Liquid chromatography-mass spectrometry general principles and instrumentation. *Journal of Chromatography A,* 1995, vol. 703 (1-2), 37-57
- **DOWDY ; WEARDEN.** Statistics for Research. John Wiley & Sons, 1983
- **ZOU ; HASTIE.** Regularization and variable selection via the elastic net. *Journal of the Royal Statistical Society, Series B,* 2005, vol. 67, 301-320
- **COLLERTON et al.** Utility of NT-proBNP as a rule-out test for left ventricular dysfunction in very old people with limiting dyspnoea: the Newcastle 85+ Study. *BMC cardiovascular disorders,* 2014, vol. 14 (1), 128
- **MUELLER-HENNESSEN et al.** A novel lipid biomarker panel for the detection of heart failure with reduced ejection fraction. *Clinical chemistry (2016): clinchem-2016,* 2016
- Methods of Enzymatic Analysis. Academic Press Inc, 1974, 1831